# EUROPEAN PATENT APPLICATION

(11) **EP 3 556 315 A1**
(43) Date of publication of application: **23.10.2019**
(21) Application number: 19169587.3
(22) Date of filing: 16.04.2019
(51) Int. Cl.: A61B 34/30, A61B 34/37, A61B 17/29, A61B 18/14

(54) **PROTECTION MEASURES FOR ROBOTIC ELECTROSURGICAL INSTRUMENTS**

(30) Priority: 17.04.2018 US 201815955226
(71) Applicant: Ethicon LLC, Guaynabo 00969 (PR)
(72) Inventor: WORRELL, Barry Christian, Cincinnati, OH 45242 (US); YOUNG, Joshua Dean, West Orange, NJ 07052 (US); TELLIO, Karalyn Radius, Cincinnati, OH 45242 (US)
(74) Representative: Bettridge, Paul Sebastian

(57) **Abstract**

A sleeve insertion tool includes an elongate cylindrical body having a closed distal end, an open proximal end, and an inner chamber extending from the proximal end toward the distal end. A jaw cavity is defined within the body and extends distally from the inner chamber, and a protective sleeve is receivable within the inner chamber and defines an aperture at a distal end thereof. An end effector with one or more jaw members is receivable within the interior and the one or more jaw members are extendable through the aperture of the protective sleeve and receivable into the jaw cavity.

## Description

### BACKGROUND

Minimally invasive surgical (MIS) instruments are often preferred over traditional open surgical devices due to reduced post-operative recovery time and minimal scarring. Laparoscopic surgery is one type of MIS procedure in which one or more small incisions are formed in the abdomen of a patient and a trocar is inserted through the incision to form a pathway that provides access to the abdominal cavity. Through the trocar, a variety of instruments and surgical tools can be introduced into the abdominal cavity. The trocar also helps facilitate insufflation to elevate the abdominal wall above the organs. The instruments and tools introduced into the abdominal cavity via the trocar can be used to engage and/or treat tissue in a number of ways to achieve a diagnostic or therapeutic effect.

Various robotic systems have recently been developed to assist in MIS procedures. Robotic systems can allow for more intuitive hand movements by maintaining natural eye-hand axis. Robotic systems can also allow for more degrees of freedom in movement by including a "wrist" joint that creates a more natural hand-like articulation. Although not necessary, the instrument's end effector can be articulated (moved) using a cable driven motion system that incorporates one or more drive cables that extend through the wrist joint. A user (e.g., a surgeon) is able to remotely operate an instrument's end effector by grasping and manipulating in space one or more controllers that communicate with a tool driver coupled to the surgical instrument. User inputs are processed by a computer system incorporated into the robotic surgical system and the tool driver responds by actuating the cable driven motion system and, more particularly, the drive cables. Moving the drive cables articulates the end effector to desired positions and configurations.

Some surgical tools, commonly referred to as electrosurgical instruments, are electrically energized. An electrosurgical instrument has a distally mounted end effector that includes one or more electrodes. When supplied with electrical energy, the end effector electrodes are able to generate heat sufficient to cut, cauterize, and/or seal tissue.

Electrosurgical instruments can be configured for bipolar or monopolar operation. In bipolar operation, current is introduced into and returned from the tissue by active and return electrodes, respectively, of the end effector. Electrical current in bipolar operation is not required to travel long distances through the patient before returning to the return electrode. Consequently, the amount of electrical current required is minimal, which greatly reduces the risk of accidental ablations and/or burns. In addition, the two electrodes are closely spaced and generally within the surgeon's field of view, which further reduces the risk of unintended ablations and burns.

In monopolar operation, current is introduced into the tissue by an active end effector electrode (alternately referred to as a "source electrode") and returned through a return electrode (e.g., a grounding pad) separately located on a patient's body. Monopolar electrosurgical instruments facilitate several surgical functions, such as cutting tissue, coagulating tissue to stop bleeding, or concurrently cutting and coagulating tissue. The surgeon can apply a current whenever the conductive portion of the instrument is in electrical proximity with the patient, permitting the surgeon to operate with monopolar electrosurgical instruments from many different angles.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of an example robotic surgical system that may incorporate some or all of the principles of the present disclosure.
FIG. 2 is a side view of an example surgical tool that may incorporate some or all of the principles of the present disclosure.
FIG. 3 illustrates potential degrees of freedom in which the wrist of FIG. 1 may be able to articulate (pivot).
FIG. 4 is an enlarged isometric view of the distal end of the surgical tool of FIG. 1.
FIGS. 5A and 5B are enlarged side views of the distal end of the surgical tool of FIG. 2.
FIGS. 6A and 6B are enlarged cross-sectional side views of the shaft adapter and the sleeve of FIG. 4, according to one or more embodiments.
FIG. 7 is a side view of another embodiment of the proximity sensor of FIGS. 6A-6B.
FIGS. 8A and 8B are enlarged side views of the distal end of another embodiment of the surgical tool of FIG. 2.
FIGS. 9A and 9B are enlarged side views of the distal end of yet another embodiment of the surgical tool of FIG. 2.
FIG. 10A is an isometric view of an example of the sleeve of FIG. 4.
FIG. 10B is a cross-sectional side view of the sleeve of FIG. 10A, according to one or more embodiments.
FIG. 10C is a cross-sectional side view of the sleeve of FIG. 10A, according to one or more additional embodiments.
FIG. 11 is an isometric view of another example of the sleeve 422 of FIG. 4.
FIG. 12 is cross-sectional side view of another example of the sleeve of FIG. 4.
FIG. 13A is an isometric view of the distal end of another embodiment of the surgical tool of FIG. 2.
FIG. 13B is a cutaway end view of the sleeve of FIG. 13A.
FIG. 14A is an enlarged side view of the distal end of another embodiment of the surgical tool of FIG. 2.
FIGS. 14B-14E depict partial isometric views of various examples of the rigid base of FIG 14A, according to one or more embodiments.
FIGS. 15A-15D are cross-sectional side views of example embodiments of coupling the rigid base of FIGS. 14A-14E to the shaft adapter of FIG. 4.
FIG. 16A is an enlarged cross-sectional side view of the shaft adapter and the sleeve of FIG. 4, according to one or more additional embodiments.
FIG. 16B is an enlarged view of a portion of FIG. 16A, as indicated by the dashed box in FIG. 16A.
FIG. 17A is an enlarged cross-sectional side view of the shaft adapter and the sleeve of FIG. 4, according to one or more additional embodiments.
FIG. 17B is an enlarged view of the expandable ring of FIG. 17A.
FIG. 17C is a cross-sectional end view of the shaft adapter of FIG. 17A, as taken along the indicated line.
FIG. 17D is a cross-sectional end view of the shaft adapter encircled by the sleeve of FIG. 17A.
FIGS. 18A and 18B are isometric assembled and exploded views, respectively, of an example insertion tool used in conjunction with the end effector of FIG. 4.
FIG. 19A is a cross-sectional side view of one example of the insertion tool of FIGS. 18A-18B.
FIG. 19B is a cross-sectional side view of another example of the insertion tool of FIGS. 18A-18B.
FIG. 20 is an isometric view of an example sleeve install assembly.
FIGS. 21A-21C are progressive isometric views of the sleeve install assembly of FIG. 20 in the process of installing the sleeve on the distal end of the surgical tool of FIGS. 2 and 4.
FIG. 22 is an enlarged cross-sectional side view of the sleeve install assembly of FIG. 20 having placed the sleeve in the assembled position.
FIG. 23 is an isometric view of the sleeve in the assembled position.
FIG. 24 is an isometric view of another example sleeve install assembly, according to one or more embodiments.
FIGS. 25A and 25B are progressive isometric views of the sleeve install assembly of FIG. 24 in the process of installing the sleeve on the distal end of the surgical tool of FIGS. 2 and 4.

### DETAILED DESCRIPTION

The present disclosure is related to robotic surgical systems that incorporate electrosurgical instruments and, more particularly, to preventing inadvertent discharge of electrical energy during operation and protecting a user from accidental cuts while installing a protective sleeve on a distal end of the electrosurgical instrument.

Embodiments discussed herein describe electrosurgical instruments or tools that use electrical energy to perform a variety of surgical procedures. The electrosurgical tools can include a wrist having a distal clevis rotatably coupled to a proximal clevis, a shaft adapter coupled to the proximal clevis, and an end effector having one or more jaw members rotatably mounted to the distal clevis. Portions of the wrist and the end effector may be insulated with a protective sleeve having a distal end and a proximal end and defining an aperture at the distal end through which the one or more jaw members protrude. When the protective sleeve moves axially from an assembled position to a migrated position, a positive indicator is provided. The positive indicator can be perceived by a user and appropriate corrective actions to halt the operation or otherwise resituate the protective sleeve may thus ensue so as to avoid electrical discharge in unintended pathways to patient tissue. In other applications, the positive indicator may be detected and a computer may be programmed to shut off electrical energy to avoid electrical discharge in unintended pathways.

Embodiments discussed herein also describe means of protecting a user during installation of a protective sleeve on the distal end of an electrosurgical tool. More specifically, a sleeve insertion tool may be provided and include an elongate cylindrical body having a closed distal end, an open proximal end, and an inner chamber extending from the proximal end toward the distal end. The protective sleeve may be received or receivable within the inner chamber. The sleeve insertion tool may be advanced over the distal end of the electrosurgical tool whereby the end effector enters the inner chamber and the one or more jaw members protrude through an aperture defined at a distal end of the protective sleeve. The one or more jaw members may be received in a jaw cavity defined within the body and extending distally from the inner chamber, and the sleeve insertion tool may be advanced proximally relative to the surgical tool to locate the protective sleeve in an assembled position on the surgical tool.

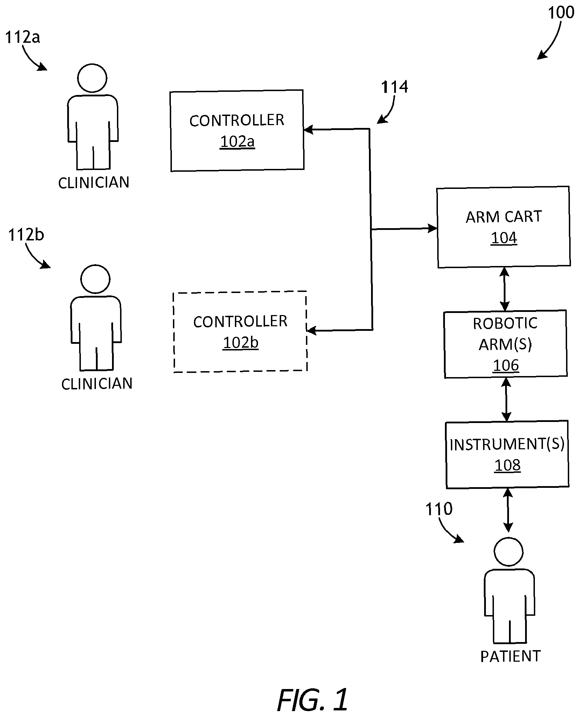

FIG. 1 is a block diagram of an example robotic surgical system 100 that may incorporate some or all of the principles of the present disclosure. As illustrated, the system 100 can include at least one master controller 102a and at least one arm cart 104, although the arm cart 104 is not necessarily required. The arm cart 104 may be mechanically and/or electrically coupled to a robotic manipulator and, more particularly, to one or more robotic arms 106 or "tool drivers". Each robotic arm 106 may include and otherwise provide a location for mounting one or more surgical tools or instruments 108 for performing various surgical tasks on a patient 110. Operation of the robotic arms 106 and instruments 108 may be directed by a clinician 112a (e.g., a surgeon) from the master controller 102a.

In some embodiments, a second master controller 102b (shown in dashed lines) operated by a second clinician 112b may also direct operation of the robotic arms 106 and instruments 108 in conjunction with the first clinician 112a. In such embodiments, for example, each clinician 102a,b may control different robotic arms 106 or, in some cases, complete control of the robotic arms 106 may be passed between the clinicians 102a,b. In some embodiments, additional arm carts (not shown) having additional robotic arms (not shown) may be utilized during surgery on a patient 110, and these additional robotic arms may be controlled by one or more of the master controllers 102a,b.

The arm cart 104 and the master controllers 102a,b may be in communication with one another via a communications link 114, which may be any type of wired or wireless telecommunications means configured to carry a variety of communication signals (e.g., electrical, optical, infrared, etc.) according to any communications protocol. In some applications, for example, there is a tower with ancillary equipment and processing cores designed to drive the robotic arms 106.

The master controllers 102a,b generally include one or more physical controllers that can be grasped by the clinicians 112a,b and manipulated in space while the surgeon views the procedure via a stereo display. The physical controllers generally comprise manual input devices movable in multiple degrees of freedom, and which often include an actuatable handle for actuating the surgical instrument(s) 108, for example, for opening and closing opposing jaws, applying an electrical potential (current) to an electrode, or the like. The master controllers 102a,b can also include an optional feedback meter viewable by the clinicians 112a,b via a display to provide a visual indication of various surgical instrument metrics, such as the amount of force being applied to the surgical instrument (i.e., a cutting instrument or dynamic clamping member).

Example implementations of robotic surgical systems, such as the system 100, are disclosed in U.S. Patent No. 7,524,320, the contents of which are incorporated herein by reference. The various particularities of such devices will not be described in detail herein beyond that which may be necessary to understand the various embodiments and forms of the various embodiments of robotic surgery apparatus, systems, and methods disclosed herein.

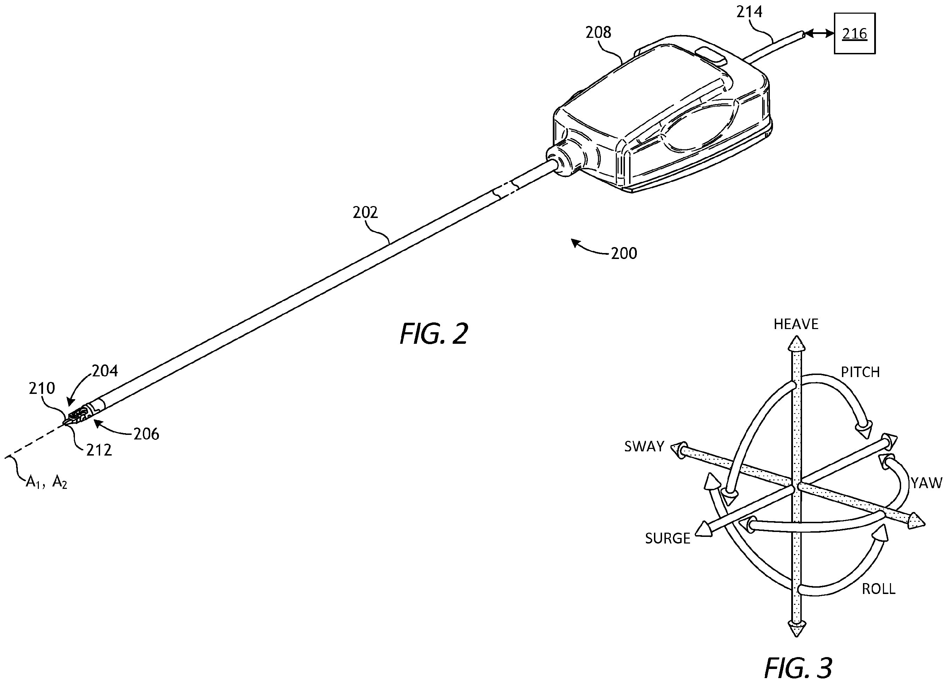

FIG. 2 is side view of an example surgical tool 200 that may incorporate some or all of the principles of the present disclosure. The surgical tool 200 may be the same as or similar to the surgical instrument(s) 108 of FIG. 1 and, therefore, may be used in conjunction with a robotic surgical system, such as the robotic surgical system 100 of FIG. 1. Accordingly, the surgical tool 200 may be designed to be releasably coupled to a tool driver included in the robotic surgical system 100. In other embodiments, however, the surgical tool 200 may be adapted for use in a manual or hand-operated manner, without departing from the scope of the disclosure.

As illustrated, the surgical tool 200 includes an elongated shaft 202, an end effector 204, a wrist 206 (alternately referred to as a "wrist joint") that couples the end effector 204 to the distal end of the shaft 202, and a drive housing 208 coupled to the proximal end of the shaft 202. In applications where the surgical tool is used in conjunction with a robotic surgical system (e.g., the robotic surgical system 100 of FIG. 1), the drive housing 208 can include coupling features that releasably couple the surgical tool 200 to the robotic surgical system.

The terms "proximal" and "distal" are defined herein relative to a robotic surgical system having an interface configured to mechanically and electrically couple the surgical tool 200 (e.g., the housing 208) to a robotic manipulator. The term "proximal" refers to the position of an element closer to the robotic manipulator and the term "distal" refers to the position of an element closer to the end effector 204 and thus further away from the robotic manipulator. Alternatively, in manual or hand-operated applications, the terms "proximal" and "distal" are defined herein relative to a user, such as a surgeon or clinician. The term "proximal" refers to the position of an element closer to the user and the term "distal" refers to the position of an element closer to the end effector 204 and thus further away from the user. Moreover, the use of directional terms such as above, below, upper, lower, upward, downward, left, right, and the like are used in relation to the illustrative embodiments as they are depicted in the figures, the upward or upper direction being toward the top of the corresponding figure and the downward or lower direction being toward the bottom of the corresponding figure.

During use of the surgical tool 200, the end effector 204 is configured to move (pivot) relative to the shaft 202 at the wrist 206 to position the end effector 204 at desired orientations and locations relative to a surgical site. The housing 208 includes (contains) various mechanisms designed to control operation of various features associated with the end effector 204 (e.g., clamping, firing, rotation, articulation, energy delivery, etc.). In at least some embodiments, the shaft 202, and hence the end effector 204 coupled thereto, is configured to rotate about a longitudinal axis A₁ of the shaft 202. In such embodiments, at least one of the mechanisms included (housed) in the housing 208 is configured to control rotational movement of the shaft 202 about the longitudinal axis A₁.

The surgical tool 200 can have any of a variety of configurations capable of performing at least one surgical function. For example, the surgical tool 200 may include, but is not limited to, forceps, a grasper, a needle driver, scissors, an electro cautery tool, a stapler, a clip applier, a hook, a spatula, a suction tool, an irrigation tool, an imaging device (e.g., an endoscope or ultrasonic probe), or any combination thereof. In some embodiments, the surgical tool 200 may be configured to apply energy to tissue, such as radio frequency (RF) energy.

The shaft 202 is an elongate member extending distally from the housing 208 and has at least one lumen extending therethrough along its axial length. In some embodiments, the shaft 202 may be fixed to the housing 208, but could alternatively be rotatably mounted to the housing 208 to allow the shaft 202 to rotate about the longitudinal axis A₁. In yet other embodiments, the shaft 202 may be releasably coupled to the housing 208, which may allow a single housing 208 to be adaptable to various shafts having different end effectors.

The end effector 204 can have a variety of sizes, shapes, and configurations. In the illustrated embodiment, the end effector 204 comprises surgical scissors that include opposing jaws 210, 212 (alternately referred to as "blades") configured to move (articulate) between open and closed positions. As will be appreciated, however, the opposing jaws 210, 212 may alternatively form part of other types of end effectors such as, but not limited to, a tissue grasper, a clip applier, a needle driver, a babcock including a pair of opposed grasping jaws, bipolar jaws (e.g., bipolar Maryland grasper, forceps, a fenestrated grasper, etc.), etc. One or both of the jaws 210, 212 may be configured to pivot at the wrist 206 to articulate the end effector 204 between the open and closed positions.

FIG. 3 illustrates the potential degrees of freedom in which the wrist 206 may be able to articulate (pivot). The wrist 206 can have any of a variety of configurations. In general, the wrist 206 comprises a joint configured to allow pivoting movement of the end effector 204 relative to the shaft 202. The degrees of freedom of the wrist 206 are represented by three translational variables (i.e., surge, heave, and sway), and by three rotational variables (i.e., Euler angles or roll, pitch, and yaw). The translational and rotational variables describe the position and orientation of a component of a surgical system (e.g., the end effector 204) with respect to a given reference Cartesian frame. As depicted in FIG. 3, "surge" refers to forward and backward translational movement, "heave" refers to translational movement up and down, and "sway" refers to translational movement left and right. With regard to the rotational terms, "roll" refers to tilting side to side, "pitch" refers to tilting forward and backward, and "yaw" refers to turning left and right.

The pivoting motion can include pitch movement about a first axis of the wrist 206 (e.g., X-axis), yaw movement about a second axis of the wrist 206 (e.g., Y-axis), and combinations thereof to allow for 360° rotational movement of the end effector 204 about the wrist 206. In other applications, the pivoting motion can be limited to movement in a single plane, e.g., only pitch movement about the first axis of the wrist 206 or only yaw movement about the second axis of the wrist 206, such that the end effector 204 moves only in a single plane.

Referring again to FIG. 2, the surgical tool 200 may also include a plurality of drive cables (obscured in FIG. 2) that form part of a cable driven motion system configured to facilitate movement and articulation of the end effector 204 relative to the shaft 202. Moving (actuating) at least some of the drive cables moves the end effector 204 between an unarticulated position and an articulated position. The end effector 204 is depicted in FIG. 2 in the unarticulated position where a longitudinal axis A₂ of the end effector 204 is substantially aligned with the longitudinal axis A₁ of the shaft 202, such that the end effector 204 is at a substantially zero angle relative to the shaft 202. Due to factors such as manufacturing tolerance and precision of measurement devices, the end effector 204 may not be at a precise zero angle relative to the shaft 202 in the unarticulated position, but nevertheless be considered "substantially aligned" thereto. In the articulated position, the longitudinal axes A₁, A₂ would be angularly offset from each other such that the end effector 204 is at a non-zero angle relative to the shaft 202.

Still referring to FIG. 2, the surgical tool 200 may be supplied with electrical power (current) via a power cable 214 coupled (permanent or detachable) to the housing 208. In other embodiments, the power cable 214 may be omitted and electrical power may be supplied to the surgical tool 200 via an internal power source, such as one or more batteries or fuel cells. For purposes of the present description, however, it will be assumed that electrical power is provided to the surgical tool 200 via the power cable 214. In either case, the surgical tool 200 may alternatively be characterized and otherwise referred to herein as an "electrosurgical instrument" capable of providing electrical energy to the end effector 204.

The power cable 214 may place the surgical tool 200 in communication with a generator 216 that supplies energy, such as electrical energy (e.g., radio frequency energy), ultrasonic energy, microwave energy, heat energy, or any combination thereof, to the surgical tool 200 and, more particularly, to the end effector 204. Accordingly, the generator 216 may comprise a radio frequency (RF) source, an ultrasonic source, a direct current source, and/or any other suitable type of electrical energy source that may be activated independently or simultaneously.

In applications where the surgical tool 200 is configured for bipolar operation, the power cable 214 will include a supply conductor and a return conductor. Current can be supplied from the generator 216 to an active (or source) electrode located at the end effector 204 via the supply conductor, and current can flow back to the generator 216 via a return conductor located at the end effector 204 via the return conductor. In the case of a bipolar tool with opposing jaws, for example, the jaws serve as the electrodes where the proximal end of the jaws are isolated from one another and the inner surface of the jaws (i.e., the area of the jaws that grasp tissue) apply the current in a controlled path through the tissue. In applications where the surgical tool 200 is configured for monopolar operation, the generator 216 transmits current through a supply conductor to an active electrode located at the end effector 204, and current is returned (dissipated) through a return electrode (e.g., a grounding pad) separately coupled to a patient's body.

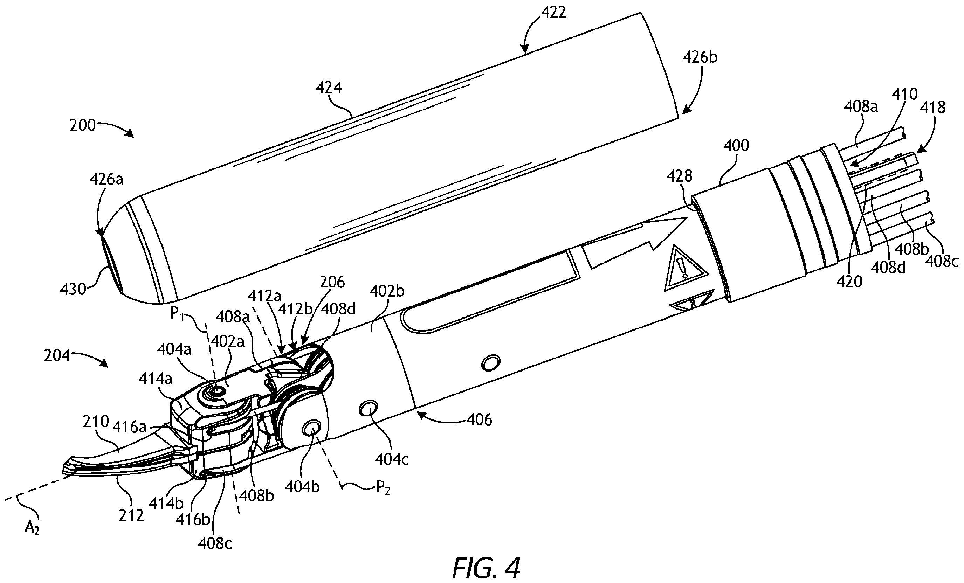

FIG. 4 is an enlarged isometric view of the distal end of the surgical tool 200 of FIG. 2. More specifically, FIG. 4 depicts enlarged views of the end effector 204 and the wrist 206, with the end effector 204 in the unarticulated position. The wrist 206 operatively couples the end effector 204 to the shaft 202 (FIG. 2). In the illustrated embodiment, however, a shaft adapter 400 may be directly coupled to the wrist 206 and otherwise interpose the shaft 202 and the wrist 206. In other embodiments, the shaft adapter 400 may be omitted and the shaft 202 may instead be directly coupled to the wrist 206, without departing from the scope of the disclosure. As used herein, the term "operatively couple" refers to a direct or indirect coupling engagement. Accordingly, the wrist 206 may be operatively coupled to the shaft 202 either through a direct coupling engagement where the wrist 206 is directly coupled to the distal end of the shaft 202, or an indirect coupling engagement where the shaft adapter 400 interposes the wrist 206 and the distal end of the shaft 202.

To operatively couple the end effector 204 to the shaft 202 (e.g., via the shaft adapter 400), the wrist 206 includes a distal clevis 402a and a proximal clevis 402b. The end effector 204 (i.e., the jaws 210, 212) is rotatably mounted to the distal clevis 402a at a first axle 404a, the distal clevis 402a is rotatably mounted to the proximal clevis 402b at a second axle 404b, and the proximal clevis 402b is coupled to a distal end 406 of the shaft adapter 400 (or alternatively the distal end of the shaft 202).

The wrist 206 provides a first pivot axis P₁ that extends through the first axle 404a and a second pivot axis P₂ that extends through the second axle 404b. The first pivot axis P₁ is substantially perpendicular (orthogonal) to the longitudinal axis A₂ of the end effector 204, and the second pivot axis P₂ is substantially perpendicular (orthogonal) to both the longitudinal axis A₂ and the first pivot axis P₁. Movement about the first pivot axis P₁ provides "yaw" articulation of the end effector 204, and movement about the second pivot axis P₂ provides "pitch" articulation of the end effector 204. In the illustrated embodiment, the jaws 210, 212 are mounted at the first pivot axis P₁, thereby allowing the jaws 210, 212 to pivot relative to each other to open and close the end effector 204 or alternatively pivot in tandem to articulate the orientation of the end effector 204.

A plurality of drive cables, shown as drive cables 408a, 408b, 408c, and 408d, extend longitudinally within a lumen 410 defined by the shaft adapter 400 (and/or the shaft 202 of FIG. 2) and pass through the wrist 206 to be operatively coupled to the end effector 204. While four drive cables 408a-d are depicted in FIG. 4, more or less than four drive cables 408a-d may be included, without departing from the scope of the disclosure.

The drive cables 408a-d form part of the cable driven motion system briefly described above, and may be referred to and otherwise characterized as cables, bands, lines, cords, wires, ropes, strings, twisted strings, elongate members, etc. The drive cables 408a-d can be made from a variety of materials including, but not limited to, metal (e.g., tungsten, stainless steel, etc.) or a polymer. Example drive cables are described in U.S. Patent Pub. No. 2015/0209965 entitled "Compact Robotic Wrist," and U.S. Patent Pub. No. 2015/0025549 entitled "Hyperdexterous Surgical System," the contents of which are hereby incorporated by reference. The lumen 410 can be a single lumen, as illustrated, or can alternatively comprise a plurality of independent lumens that each receive one or more of the drive cables 408a-d.

The drive cables 408a-d extend proximally from the end effector 204 to the drive housing 208 (FIG. 2) where they are operatively coupled to various actuation mechanisms or devices housed (contained) therein to facilitate longitudinal movement (translation) of the drive cables 408a-d within the lumen 410. Selective actuation of all or a portion of the drive cables 408a-d causes the end effector 204 (e.g., one or both of the jaws 210, 212) to articulate (pivot) relative to the shaft 202. More specifically, selective actuation causes a corresponding drive cable 408a-d to translate longitudinally within the lumen 410 and thereby cause pivoting movement of the end effector 204. One or more drive cables 408a-d, for example, may translate longitudinally to cause the end effector 204 to articulate (e.g., both of the jaws 210, 212 angled in a same direction), to cause the end effector 204 to open (e.g., one or both of the jaws 210, 212 move away from the other), or to cause the end effector 204 to close (e.g., one or both of the jaws 210, 212 move toward the other).

Moving the drive cables 408a-d can be accomplished in a variety of ways, such as by triggering an associated actuator or mechanism operatively coupled to or housed within the drive housing 208 (FIG. 2). Moving a given drive cable 408a-d constitutes applying tension (i.e., pull force) to the given drive cable 408a-d in a proximal direction, which causes the given drive cable 408a-d to translate and thereby cause the end effector 204 to move (articulate) relative to the shaft 202.

The wrist 206 includes a first plurality of pulleys 412a and a second plurality of pulleys 412b, each configured to interact with and redirect the drive cables 408a-d for engagement with the end effector 204. The first plurality of pulleys 412a is mounted to the proximal clevis 402b at the second axle 404b and the second plurality of pulleys 412b is also mounted to the proximal clevis 402b but at a third axle 404c located proximal to the second axle 404b. The first and second pluralities of pulleys 412a,b cooperatively redirect the drive cables 408a-d through an "S" shaped pathway before the drive cables 408a-d are operatively coupled to the end effector 204.

In at least one embodiment, one pair of drive cables 408a-d is operatively coupled to each jaw 210, 212 and configured to "antagonistically" operate the corresponding jaw 210, 212. In the illustrated embodiment, for example, the first and second drive cables 408a,b are coupled with a connector (not shown) at the first jaw 210, and the third and fourth drive cables 408c,d are coupled with a connector (not shown) at the second jaw 212. Consequently, actuation of the first drive cable 408a pivots the first jaw 210 about the first pivot axis P₁ toward the open position, and actuation of the second drive cable 408b pivots the first jaw 210 about the first pivot axis P₁ in the opposite direction and toward the closed position. Similarly, actuation of the third drive cable 408c pivots the second jaw 212 about the first pivot axis P₁ toward the open position, while actuation of the fourth drive cable 408d pivots the second jaw 212 about the first pivot axis P₁ in the opposite direction and toward the closed position.

Accordingly, the drive cables 408a-d may be characterized or otherwise referred to as "antagonistic" cables that cooperatively (yet antagonistically) operate to cause relative or tandem movement of the first and second jaws 210, 212. When the first drive cable 408a is actuated (moved), the second drive cable 408b naturally follows as coupled to the first drive cable 408a, and when the third drive cable 408c is actuated, the fourth drive cable 408d naturally follows as coupled to the third drive cable 408c, and vice versa.

The end effector 204 further includes a first jaw holder 414a and a second jaw holder 414b laterally offset from the first jaw holder 414a. The first jaw holder 414a is mounted to the first axle 404a and configured to receive and seat the first jaw 210 such that movement (rotation) of the first jaw holder 414a about the first pivot axis P₁ correspondingly moves (rotates) the first jaw 210. The first jaw holder 414a may also provide and otherwise define a first pulley 416a configured to receive and seat one or more drive cables, such as the first and second drive cables 408a,b to effect such movement (rotation). The second jaw holder 414b is similarly mounted to the first axle 404a and is configured to receive and seat the second jaw 212 such that movement (rotation) of the second jaw holder 414b about the first pivot axis P₁ correspondingly moves (rotates) the second jaw 212. The second jaw holder 414b may also provide and otherwise define a second pulley 416b configured to receive and seat one or more drive cables, such as the third and fourth drive cables 408c,d, to effect such movement (rotation).

The term "jaw holder," as used herein, is intended to apply to a variety of types of end effectors having opposing jaws or blades that are movable relative to one another. In the illustrated embodiment, the jaws 210, 212 comprise opposing scissor blades of a surgical scissors end effector. Accordingly, the jaw holders 414a,b may alternately be referred to as "blade holders". In other embodiments, however, the jaws 210, 212 may alternatively comprise opposing jaws used in a grasper end effector, or the like, and the term "jaw holder" similarly applies, without departing from the scope of the disclosure. Moreover, the term "holder" in "jaw holder" may be replaced with "mount," "drive member," or "actuation member."

The surgical tool 200 may also include an electrical conductor 418 that supplies electrical energy to the end effector 204, thereby converting the surgical tool 200 into an "electrosurgical instrument". Similar to the drive cables 408a-d, the electrical conductor 418 may extend longitudinally within the lumen 410. In some embodiments, the electrical conductor 418 and the power cable 214 (FIG. 2) may comprise the same structure. In other embodiments, however, the electrical conductor 418 may be electrically coupled to the power cable 214, such as at the drive housing 208 (FIG. 2). In yet other embodiments, the electrical conductor 418 may extend to the drive housing 208 where it is electrically coupled to an internal power source, such as batteries or fuel cells.

In some embodiments, the electrical conductor 418 may comprise a wire. In other embodiments, however, the electrical conductor 418 may comprise a rigid or semi-rigid shaft, rod, or strip (ribbon) made of a conductive material. In some embodiments, the electrical conductor 418 may be partially covered with an insulative covering 420 (shown in dashed lines) made of a non-conductive material. The insulative covering 420, for example, may comprise a plastic applied to the electrical conductor 418 via heat shrinking, but could alternatively be any other non-conductive material.

In operation, the end effector 204 may be configured for monopolar or bipolar operation, without departing from the scope of the disclosure. Electrical energy is transmitted by the electrical conductor 418 to the end effector 204, which acts as an active (or source) electrode. In at least one embodiment, the electrical energy conducted through the electrical conductor 418 may comprise radio frequency ("RF") energy exhibiting a frequency between about 100 kHz and 1 MHz. The RF energy causes ultrasonic agitation or friction, in effect resistive heating, thereby increasing the temperature of target tissue. Accordingly, electrical energy supplied to the end effector 204 is converted to heat and transferred to adjacent tissue to cut, cauterize, and/or coagulate the tissue (dependent upon the localized heating of the tissue), and thus may be particularly useful for sealing blood vessels or diffusing bleeding.

The surgical tool 200 may further include a protective sleeve 422 configured to insulate various live (energized) portions of the end effector 204 (including the wrist 206), and thereby protect the patient from stray electrical discharge during operation. As illustrated, the sleeve 422 may comprise an elongate and generally cylindrical body 424 having a first or distal end 426a and a second or proximal end 426b opposite the distal end 426a. The body 424 may be sized to extend over portions of the end effector 204, the wrist 206, and the shaft adapter 400 (or alternatively the shaft 202 when the shaft adapter 400 is omitted). When the sleeve 422 is properly positioned for use, the jaw members 210, 212 protrude out an aperture 430 defined in the distal end 426a of the body 424 and the proximal end 426b engages or comes into close contact with a radial shoulder 428 defined on the shaft adapter 400. When the sleeve 422 is properly positioned (installed), electrical current can only be conducted to patient tissue as intended at the exposed jaw members 210, 212.

The sleeve 422 may be assembled onto the tool 200 within the sterile field before surgery and removed before cleaning the tool 200. The sleeve 422 must be properly installed to mitigate electrical discharge in unintended pathways, and the responsibility for proper installation is often left to the various scrub nurses on hand in an operating room. One challenge is error proofing proper installation of the sleeve 422 and ensuring that the sleeve 422 is properly positioned for use. Embodiments of the present disclosure include several possible means of ensuring proper assembly (installation) of the sleeve 422.

Moreover, the sleeve 422 is generally made of a flexible material and installed via an interference fit between the inner radial surface of the sleeve 422 and the outer radial surfaces of the end effector 204, the wrist 206, and/or the shaft adapter 400. The flexibility of the sleeve 422 allows the wrist 206 to articulate during use. As the wrist 206 articulates, however, the sleeve 422 may have a tendency to creep axially, which results in the proximal end 426b separating from the radial shoulder 428 and increasing the likelihood of electrical discharge in unintended pathways. Embodiments described herein provide a positive indicator that the sleeve 422 has moved from its properly assembled position. Consequently, a user (e.g., a surgeon) may be alerted that electrical discharge in unintended pathways to the patient tissue may potentially ensue, thus prompting action to properly resituate the sleeve 422 if warranted.

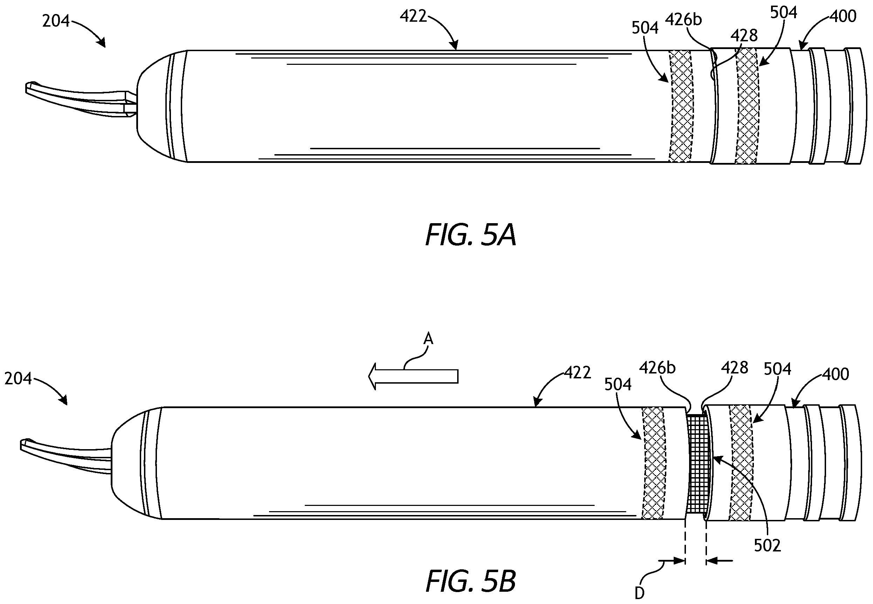

FIGS. 5A and 5B are enlarged side views of the distal end of the surgical tool 200 of FIG. 2. More specifically, FIG. 5A depicts the sleeve 422 in a first or "assembled" position, where the sleeve 422 is properly positioned on the end effector 204 for operation, and FIG. 5B depicts the sleeve 422 in a second or "migrated" position, where the sleeve 422 has moved (migrated) axially from the assembled position and relative to at least one of the end effector 204 and the shaft adapter 400.

In the assembled position shown in FIG. 5A, the sleeve 422 is located in a position that properly insulates the end effector 204 and otherwise mitigates electrical discharge in unintended pathways to patient tissue. In the illustrated embodiment, for example, the sleeve 422 may be considered to be in the assembled position when the proximal end 426b of the sleeve 422 abuts or comes into close contact with the radial shoulder 428 of the shaft adapter 400. In other embodiments, however, the sleeve 422 may be considered to be in the assembled position when located at other axial locations relative to one or both of the end effector 204 and the shaft adapter 400, as long as the sleeve 422 operates to mitigate electrical discharge in unintended pathways to patient tissue. Consequently, variations in the design and construction of the shaft adapter 400 and the sleeve 422 (and how they interrelate) are contemplated herein and considered to fall within the scope of the disclosure as long as the sleeve 422 achieves its mitigating (protective) purpose.

In the migrated position shown in FIG. 5B, the sleeve 422 has migrated (crept) axially in the distal direction A from the assembled position (however, the migrated position may alternatively comprise moving proximally, as described below). The migrated position of the sleeve 422 is not limited to a particular distance or location offset from the assembled position. Rather, the migrated position may comprise any axial position relative to at least one of the end effector 204 and the shaft adapter 400 where the sleeve 422 may be unable to properly prevent (mitigate) electrical discharge in unintended pathways to patient tissue. In the illustrated embodiment, for example, the sleeve 422 is shown moved to the migrated position by separating the proximal end 426b of the sleeve 422 from the radial shoulder 428 by a distance D. In other words, the sleeve 422 has moved from the assembled position by a distance of D. The distance D can be any predetermined distance or length that the sleeve 422 travels (migrates) before action is taken (e.g., an alert sent, power shut off, etc.), and will depend largely upon the design of the surgical tool 200 (FIG. 2) and the application.

In at least one embodiment, the distance D may correspond to an offset distance from a location where an exposed charge point exists. More specifically, in some embodiments, the distance D may correspond to a distance of about six millimeters from the exposed charge point. Once the sleeve 422 has crept (migrated) to the distance D, only about six millimeters of insulating sleeve 422 material exists between the exposed charge point and the end of the sleeve 422.

The end effector 204 is typically introduced into a patient cavity simultaneously with a light source and a surgical camera. The light source illuminates the interior of the patient cavity and the camera provides a live feed that allows the user (e.g., a surgeon) to observe and conduct the operation in real-time via interconnected monitors or other visual aids. In some embodiments, as the sleeve 422 creeps toward the migrated position and otherwise away from the assembled position during operation, an indicator 502 may become exposed and thereby provide a positive indicator that the sleeve 422 has transitioned from the assembled position.

The indicator 502 may comprise a visible component that may be optically detected, observed, perceived, or registered through the live camera feed. In some embodiments, the user may physically observe the indicator 502 via the camera feed and thereby be alerted in real-time when the sleeve 422 has moved to or is progressing toward the migrated position. In other embodiments, the surgical camera may comprise a digital camera programmed to detect or register a preselected (predetermined) indicator 502 and may either alert the user of sleeve 422 movement or autonomously cut off power to the tool to prevent electrical discharge in unintended pathways.

Alternatively, when the indicator 502 is not visible or detectable via the camera feed, that may be a positive indicator that the sleeve 422 is safely situated in the assembled position for operation. Accordingly, prior to commencing an operation, the camera may pan out to ensure that the sleeve 422 is properly installed, which can be verified by the absence of the indicator 502.

The indicator 502 may comprise, but is not limited to, a color, a pattern, electromagnetic radiation, a photoluminescent or phosphorescent material or substance, a reflective surface or material, or any combination thereof. In the illustrated embodiment, the indicator 502 forms part of or is otherwise coupled (attached) to the outer radial surface of the shaft adapter 400. In other embodiments, however, the indicator 502 may form part of or may otherwise be coupled (attached) to the outer radial surface of the end effector 204 or the wrist 206 (FIG. 4), without departing from the scope of the disclosure.

In embodiments where the indicator 502 is a color, the color may comprise a paint, a covering, or an overlay of any tone or grade capable of being perceived by or transmitted through the camera. In such embodiments, a user need only observe the preselected color of the indicator 502 through the camera feed during operation to know that the sleeve 422 has moved to/toward the migrated position. In other embodiments, however, the camera may be programmed to detect a preselected (predetermined) color and may communicate with a computer system that sends an alert (audible, visual, etc.) to the user when the color is detected. Alternatively, the computer system may be programmed to autonomously cut power to the end effector 204 when the color is detected to prevent electrical discharge in unintended pathways. Cutting the power does not necessarily remove all power to the end effector 204, but may be limited to cutting power via the electrical conductor 418 (FIG. 4).

In embodiments where the indicator 502 is a pattern, the pattern may comprise any perceivable design or configuration recognizable by the human eye or detectable by the camera. In some embodiments, for example, the pattern may comprise a checker board design or the like.

In embodiments where the indicator 502 comprises electromagnetic radiation, the indicator 502 may be configured to emit the electromagnetic radiation upon becoming exposed. Example forms of electromagnetic radiation include, but are not limited to, radio waves, microwave radiation, infrared and near-infrared radiation, visible light, ultraviolet light, X-ray radiation or gamma ray radiation. Visible light, for example, may be perceivable by the user through the camera feed. In such embodiments, the indicator 502 may comprise one or more LED lights or the like that become exposed upon moving the sleeve 422 to the migrated position. In some embodiments, the visible light may flash in a specific frequency that is picked up by the surgical camera thus prompting disablement of the power generator. In other embodiments, the LED lights might emit infrared or near-infrared radiation that only the surgical camera can detect.

In embodiments where the indicator 502 comprises a highly reflective covering, the indicator 502 may include a reflective paint or a surface, such as a highly polished surface. When the light from the light source used in the operation shines on the indicator 502, the reflected light shines brightly in the camera. Alternatively, the indicator 502 may comprise a specific photoluminescent or phosphorescent material/substance capable of being observed by human eyes or detected by the surgical camera.

Referring to both FIGS. 5A and 5B, in some embodiments, the surgical tool 200 may further include an over-assembled indicator 504 (shown in dashed lines). The over-assembled indicator 504 may be similar to the indicator 502 except that it is used to help the user detect when the sleeve 422 may be advanced (or migrate) proximally past the assembled position, which may comprise another type of migrated position but in the proximal direction. The sleeve 422 may move proximally past the assembled position during operation as a result of articulation of the wrist 206 (FIG. 4), or though advancing the sleeve 422 too far during installation. The camera may be configured to monitor the location of the over-assembled indicator 504, and when the sleeve 422 occludes the over-assembled indicator 504, the camera may communicate with a computer system that sends an alert (audible, visual, etc.). Accordingly, non-detection of the over-assembled indicator 504 may comprise a positive indicator that the protective sleeve 422 has moved from the assembled position to the migrated position in the proximal direction.

Still referring to both FIGS. 5A-5B, in some embodiments, the surgical tool 200 may include a sleeve indicator 506. The sleeve indicator 506 may be similar to the indicator 502, but may comprise the sleeve 422 itself or a visible component of the sleeve 422 that may be optically detected, observed, perceived, or registered through the live camera feed. The camera may be configured to monitor the position of the sleeve 422 by detecting the sleeve indicator 506 at a specific location on the sleeve 422. As long as the sleeve indicator 506 is detected, the user can be assured that the sleeve 422 is properly positioned in the assembled position. When the sleeve indicator 506 is no longer detectable at that location, because the sleeve 422 has moved toward the migrated position, the camera may communicate with a computer system that sends an alert (audible, visual, etc.). Alternatively, the computer system may be programmed to autonomously cut power to the end effector 204 when the sleeve indicator 506 is not detected to prevent electrical discharge in unintended pathways. Accordingly, non-detection of the sleeve indicator 506 may comprise a positive indicator that the protective sleeve 422 has moved from the assembled position to the migrated position. Moreover, during assembly, if the sleeve indicator 506 is not detectable at a predetermined location as determined by the camera, the end effector 204 may not be activated.

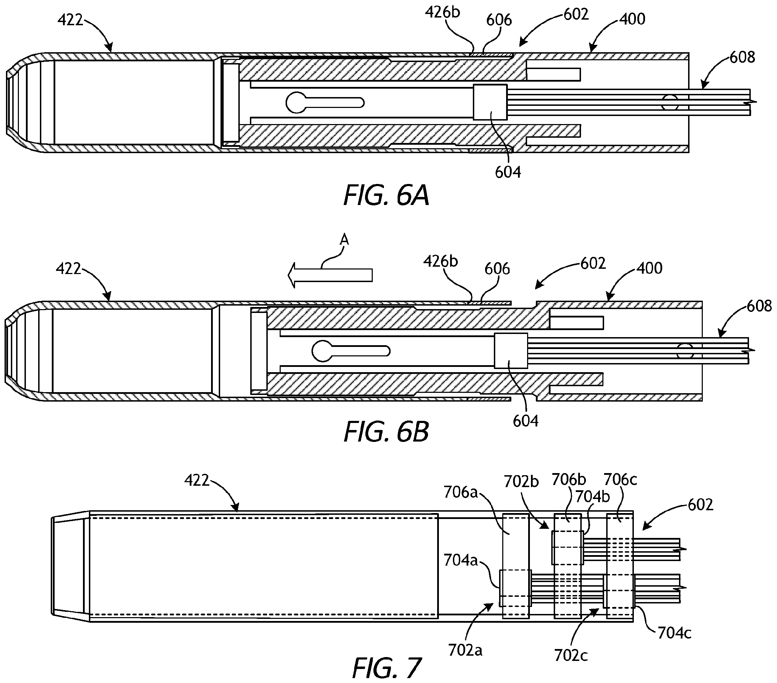

FIGS. 6A and 6B are enlarged cross-sectional side views of the shaft adapter 400 and the sleeve 422 of FIG. 4, according to one or more embodiments. More specifically, FIG. 6A depicts the sleeve 422 in the assembled position, and FIG. 6B depicts the sleeve 422 in the migrated position after having moved axially in the distal direction A relative to the shaft adapter 400.

In the illustrated embodiment, a proximity sensor 602 is incorporated to provide a positive indicator when the sleeve 422 has moved (migrated) from the assembled position. More particularly, the proximity sensor 602 may include a hall effect sensor 604 and a metallic component 606 coupled to the sleeve 422. The hall effect sensor 604 may be positioned within and otherwise extended into the interior of the shaft adapter 400. Alternatively, or in addition thereto, the hall effect sensor 604 may be extended into the interior of the sleeve 422. In either scenario, the hall effect sensor 604 may be positioned in proximity to the metallic component 606 to detect the presence or non-presence thereof. In some embodiments, the hall effect sensor 604 may be programmed to detect a degree of presence or non-presence when the sleeve 422 is partially moved. The hall effect sensor 604 may comprise a transducer that includes one or more communication lines 608 that extend from the drive housing 208 (FIG. 2) to the hall effect sensor 604 and facilitate communication therebetween.

The metallic component 606 may be made of any magnetic metal capable of being detected by the hall effect sensor 604. In some embodiments, the metallic component 606 may be molded into the inner radial surface of the sleeve 422 at or near the proximal end 426b. In other embodiments, the metallic component 606 may be coupled to the inner radial surface of the sleeve 422 using an adhesive or some type of mechanical attachment (e.g., mechanical fasteners, a snap fit engagement, an interference fit, etc.). In other embodiments, the metallic component 606 may be coupled to the proximal end 426b of the sleeve 422, without departing from the scope of the disclosure. In some embodiments, the metallic component 606 may comprise an annular ring and extend about the entire circumference of the sleeve 422. In other embodiments, however, the metallic component 606 might extend only partially about the circumference of the sleeve 422, without departing from the scope of the disclosure.

When the sleeve 422 is in the assembled position, as shown in FIG. 6A, the hall effect sensor 604 may be generally axially aligned with the metallic component 606. Alternatively, the hall effect sensor 604 may simply be able to detect the presence of the metallic component 606. When the sleeve 422 transitions to the migrated state, as shown in FIG. 6B, the metallic component 606 correspondingly moves in the distal direction A and axially away from the hall effect sensor 604. As the metallic component 606 moves in the distal direction A, the hall effect sensor 604 detects this movement and provides an alert (audible, visual, etc.) to the user. In some embodiments, the user may react to the alert and make adjustments to the sleeve 422. In other embodiments, once the metallic component 606 moves a predetermined distance from the sensor 604, the sensor 604 may send a signal to a computer system programmed to autonomously cut off power to the tool to prevent electrical discharge in unintended pathways.

FIG. 7 is a side view of another embodiment of the proximity sensor 602 of FIGS. 6A-6B. More specifically, the proximity sensor 602 of FIG. 7 incorporates a plurality of proximity sensors, shown as a first proximity sensor 702a, a second proximity sensor 702b, and a third proximity sensor 702c. The proximity sensors 702a-c may cooperatively provide an indication when the sleeve 422 has moved (migrated) from the assembled position.

The first proximity sensor 702a includes a first hall effect sensor 704a and a first metallic component 706a, the second proximity sensor 702b includes a second hall effect sensor 704b and a second metallic component 706b, and the third proximity sensor 702c includes a third hall effect sensor 704c and a third metallic component 706c. The hall effect sensors 704a-c and the metallic components 706a-c may be similar to the hall effect sensor 604 and the metallic component 606 of FIGS. 6A-6B and, therefore, will not be described again.

When the sleeve 422 is in the assembled position, the hall effect sensors 704a-c may be axially aligned with the corresponding metallic components 706a-c. Alternatively, the hall effect sensors 704a-c may simply be able to detect the presence of the metallic components 706a-c. When the sleeve 422 transitions to the migrated state, however, the metallic components 706a-c correspondingly move in the same direction and axially away from the associated hall effect sensors 704a-c. As the metallic components 706a-c move distally, the hall effect sensors 704a-c detect this movement and provide an alert (audible, visual, etc.) to the user of such movement.

Having the plurality of proximity sensors 702a-c may provide redundancy in the event one of the proximity sensors 702a-c malfunctions. In other applications, however, the plurality of proximity sensors 702a-c may be used to provide different levels or types of warning or alerts. For instance, if the first metallic component 706a is detected by the second hall effect sensor 704b, an alert or warning may be issued by the second sensor 704b that the sleeve 422 is moving distally to the migrated position. If the first metallic component 706a is detected by the third hall effect sensor 704c, however, the third hall effect sensor 704c may send a stronger alert or alternatively cut the power to the tool. Moreover, in some embodiments, the proximity sensors 702a-c may be programmed to detect when the sleeve 422 has migrated proximally. Proximal migration can occur in an instance of misassembly where the sleeve 422 is pushed too far.

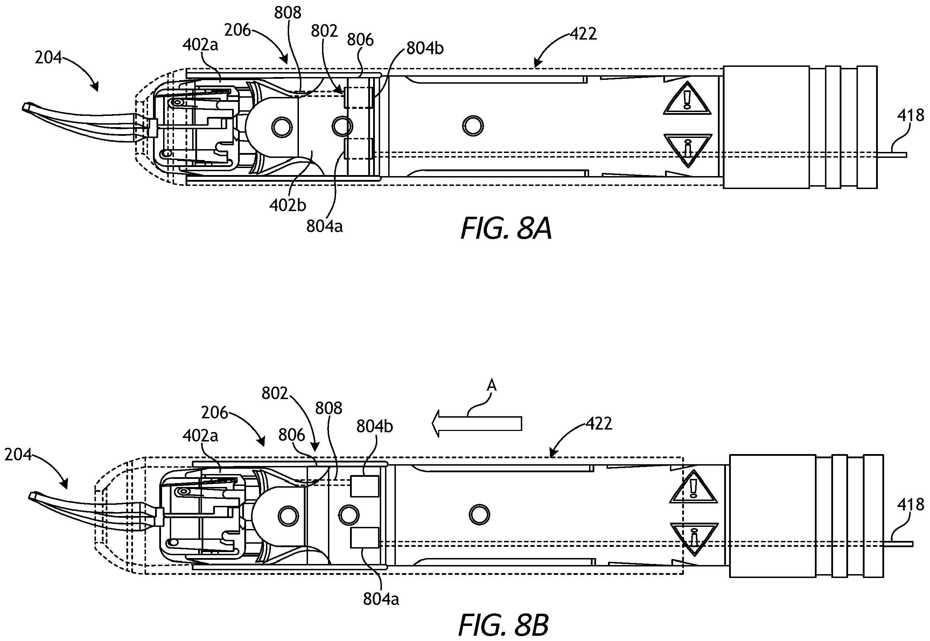

FIGS. 8A and 8B are enlarged side views of the distal end of another embodiment of the surgical tool 200 of FIG. 2. More specifically, FIG. 8A depicts the sleeve 422 in the assembled position, and FIG. 8B depicts the sleeve 422 in the migrated position after having moved axially in the distal direction A relative to the shaft adapter 400.

In the illustrated embodiment, a continuity connection 802 may be provided at or near the end effector 204 to facilitate the transmission of electrical energy to the end effector 204. As illustrated, the continuity connection 802 may include a pair of contacts 804a and 804b and an associated contact strip 806. The contacts 804a,b may be coupled to the wrist 206, such as at the proximal clevis 402b, and offset from each other such that electrical communication between the contacts 804a,b is prevented independently. The electrical conductor 418 may extend to the first contact 804a and provide electrical energy thereto. A second electrical conductor 808 may extend between the second contact 804b and the distal clevis 402a such that electrical energy provided to the second contact 804b may be transmitted to the distal clevis 402a to energize the end effector 204.

The contact strip 806 may be configured to complete the electrical circuit between the contacts 804a,b. Accordingly, the contact strip 806 may be made of an electrically conductive material (e.g., a metal) and coupled to the sleeve 422, such as being secured to or overmolded onto the inner radial surface of the sleeve 422. The contact strip 806 may exhibit an angular length sufficient to extend between the contacts 804a,b when the sleeve 422 is in the assembled position and thereby place the contacts 804a,b in electrical communication. In some embodiments, the contact strip 806 may comprise an annular ring extending about the entire inner circumference of the sleeve 422. In other embodiments, however, the contact strip 806 may be long enough to complete the continuity connection 802 between the contacts 804a,b.

When electrical energy is supplied to the end effector, the continuity connection 802 essentially provides a positive indicator that the sleeve 422 is in the assembled position. More particularly, when the sleeve 422 is in the assembled position, as shown in FIG. 8A, the continuity connection 802 is complete and electrical energy supplied by the electrical conductor 418 can be transmitted between the contacts 804a,b via the contact strip 806, and to the end effector 204 via the second electrical conductor 808. In contrast, when the sleeve 422 is in the migrated position, as shown in FIG. 8B, the continuity connection 802 is shorted out and electrical energy is cut-off from the end effector 204. More specifically, as the contact strip 806 moves distally in the direction A and out of electrical contact with the contacts 804a,b, the continuity connection 802 short circuits and the end effector 204 ceases to be energized.

As will be appreciated, this embodiment may also prove advantageous in shorting the electrical circuit until the sleeve 422 is properly assembled and otherwise placed in the assembled position. Consequently, the end effector 204 will not be energized for use unless the sleeve 422 is in the assembled position.

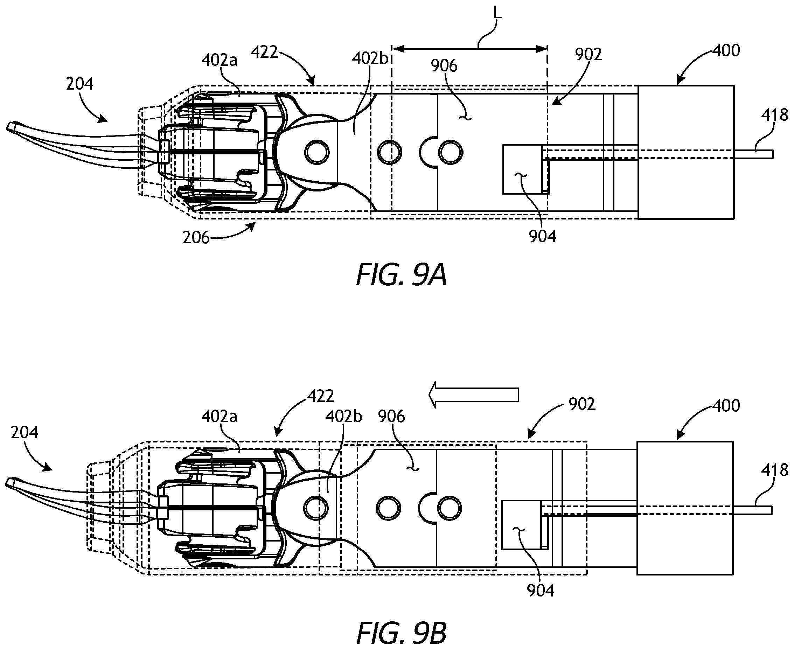

FIGS. 9A and 9B are enlarged side views of the distal end of another embodiment of the surgical tool 200 of FIG. 2. More specifically, FIG. 9A depicts the sleeve 422 (shown in phantom) in the assembled position, and FIG. 9B depicts the sleeve 422 in the migrated position after having moved axially in the distal direction A relative to the end effector 204 and the shaft adapter 400.

Similar to the embodiment of FIGS. 8A-8B, the embodiment of FIGS. 9A-9B provides a continuity connection 902 at or near the end effector 204 to facilitate the transmission of electrical energy to the end effector 204 and thereby provide a positive indicator that the sleeve 422 is in the assembled position. In the illustrated embodiment, the continuity connection 902 includes a contact 904 and an associated contact strip 906. The contact 904 may be coupled to the shaft adapter 400 and exposed such that it can be placed in electrical communication with the contact strip 906. In the illustrated embodiment, for example, the contact 904 may extend through the wall of the shaft adapter 400 or otherwise be arranged on the outer radial surface thereof. The electrical conductor 418 may extend to the contact 904 and provide electrical energy thereto.

The contact strip 906 may be made of an electrically conductive material (e.g., a metal) and coupled to the sleeve 422. In some embodiments, for example, the contact strip 906 may be secured to or overmolded onto the inner radial surface of the sleeve 422. The contact strip 906 may exhibit an axial length L sufficient to extend between the contact 904 and at least the proximal clevis 402b when the sleeve 422 is in the assembled position, and thereby place the contact 904 in electrical communication with the end effector 204 via the electrically conductive wrist 206. In at least one embodiment, the axial length L may be long enough to extend to the distal clevis 402a. In some embodiments, the contact strip 906 may comprise an annular ring (sleeve) extending about the entire inner circumference of the sleeve 422. In other embodiments, however, the contact strip 906 may be in any form or shape and merely long enough to complete the continuity connection 902 between the contact 904 and the end effector 204.

When the sleeve 422 is in the assembled position, as shown in FIG. 9A, the continuity connection 902 is complete and electrical energy supplied by the electrical conductor 418 can be transmitted between the contact 904 and the end effector 204 (e.g., the proximal clevis 402b) via the contact strip 906. In contrast, when the sleeve 422 is in the migrated position, as shown in FIG. 9B, the contact strip 906 moves distally in the direction A and out of electrical contact with the contact 904, thereby short circuiting the continuity connection 902. Accordingly, once the sleeve 422 moves to the migrated position, the end effector 204 ceases to be energized.

As will be appreciated, this embodiment may also prove advantageous in shorting the electrical circuit until the sleeve 422 is properly assembled and otherwise placed in the assembled position. Consequently, the end effector 204 will not be energized for use unless the sleeve 422 is in the assembled position.

In some embodiments, the contact strip 906 may be made of a silicone composite material, but could also be made of a polymer or an elastomer, without departing from the scope of the disclosure. In such embodiments, for example, the contact strip 906 may comprise a conductive material embedded in a silicone matrix. The conductive material may comprise, but is not limited to, silver coated glass beads, graphite, carbon black, metallic filaments, a plating, a metal (e.g., aluminum or copper), or any combination thereof. The conductive material allows the contact strip 906 to conduct electrical energy, as discussed above. Moreover, in such embodiments, the contact strip 906 made of the silicone composite material may be flexible, whereas a purely metallic contact strip 906 would stiffen the sleeve 422.

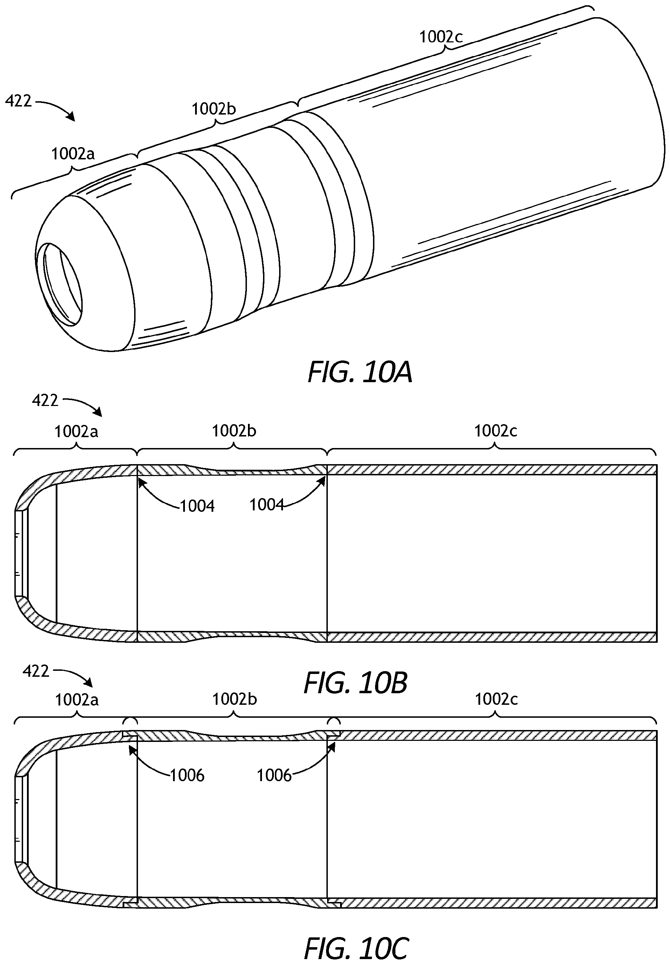

FIG. 10A is an isometric view of an example of the sleeve 422 of FIG. 4, according to one or more embodiments. As illustrated, the sleeve 422 may comprise a plurality of discrete sections, shown as a first section 1002a, a second section 1002b, and a third section 1002c, where the second section 1002b generally interposes the first and third sections 1002a,c. The first section 1002a may be made of a first material having a first coefficient of elasticity v₁, the second section 1002b may be made of a second material having a second coefficient of elasticity v₂, and the third section 1002c may be made of a third material having a third coefficient of elasticity v₃.

In some embodiments, the second coefficient of elasticity v₂ may be greater than the first coefficient of elasticity v₁, and the first coefficient of elasticity v₁ may be greater than the third coefficient of elasticity v₃. In other words, v₂ > v₁ > v₃. In such embodiments, the material of the second section 1002b may be made of a highly flexible material including, but not limited to, thermoplastic polyurethane (TPU), nitrile rubber, polyisoprene, and any elastomer that exhibits a high strain capacity. The material of the first section 1002a may be made of a flexible material including, but not limited to, silicone. Lastly, the material of the third section 1002c may be made of a stiffer material including, but not limited to, polyether ether ketone (PEEK), a high modulus of elasticity TPU, polycarbonate, or any combination thereof.

Having the second section 1002b made of a material that is more flexible than the others may prove advantageous since the wrist 206 (FIG. 4) is generally located beneath the second section 1002b. Consequently, the sleeve 422 will be flexed to a greater magnitude at the second section 1002b as opposed to the first and third sections 1002a,c during operation as the wrist 206 articulates to move the end effector 204 (FIG. 2). Moreover, having the third section 1002c made of a stiffer material may prove advantageous in providing a positive hard stop when the sleeve 422 is placed in the assembled position e.g., against the radial shoulder 428 (FIG. 4) of the shaft adapter 400 (FIG. 4).

FIG. 10B is a cross-sectional side view of the sleeve 422 of FIG. 10A, according to one or more embodiments. As illustrated, some or all of the first, second, and third sections 1002a-c may be adjoined at respective butt joints 1004 (two shown), where the adjacent axial ends of each section 1002a-c are secured using, for example, an adhesive or sonic welding.

FIG. 10C is a cross-sectional side view of the sleeve 422 of FIG. 10A, according to one or more additional embodiments. As illustrated, the second section 1002b may be coupled to one or both of the first and third sections 1002a,c via an overlapping lap joint 1006 (two shown). The lap joint(s) 1006 may secure the adjacent sections 1002a-c together via a variety of attachment means including, but not limited to, an interference fit, an adhesive, sonic welding, a threaded engagement, or any combination thereof.

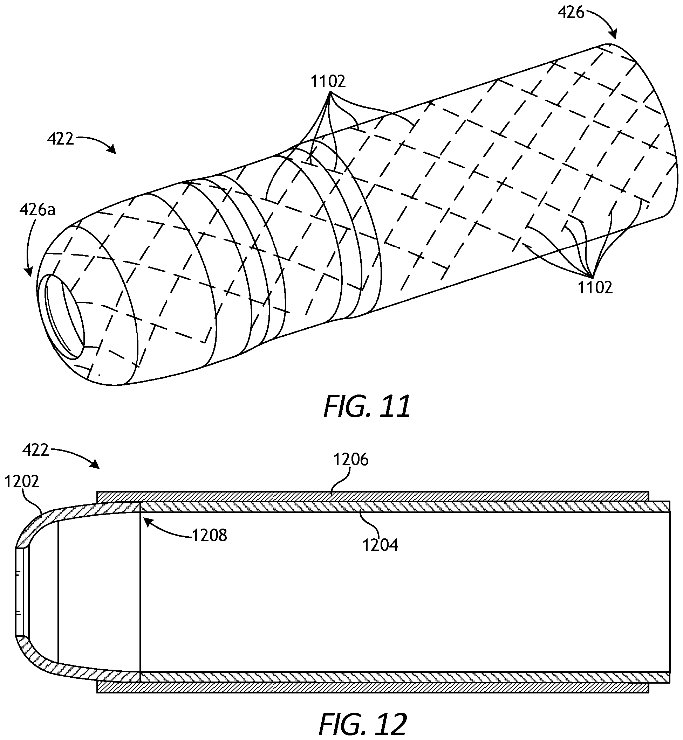

FIG. 11 is an isometric view of another example of the sleeve 422 of FIG. 4, according to one or more embodiments. As illustrated, the sleeve 422 may be reinforced with and otherwise include a plurality of filaments 1102 that act as material strengthening additives. The filaments 1102 may comprise, for example, braided or non-braided polymer strands, metal strands, elastomer strands, liquid crystal polymer (LCD) monofilaments, or any combination thereof. In some embodiments, the sleeve 422 may be overmolded or co-extruded with the filaments 1102, and the filaments 1102 may be positioned or otherwise configured to reinforce the main material of the sleeve 422. In some embodiments, the filaments 1102 may be arranged in a weave pattern or a fabric-like construction similar to silicone coated fabrics.

Including the filaments 1102 in the construction of the sleeve 422 may prove advantageous for a variety of reasons. For example, the filaments 1102 may help create a more puncture-resistant sleeve 422, but with sufficient flexibility to allow the end effector 204 (FIG. 4) to articulate as needed. Moreover, the filaments 1102 give the sleeve 422 an increased level of rigidity that allows the sleeve 422 to be axially extended onto the end effector 204, while exhibiting sufficient flexibility to allow the wrist 206 (FIG. 4) to move. More specifically, the filaments 1102 decrease compressibility, but allow the sleeve 422 to retain its flexibility.

In some embodiments, the density of the filaments 1102 may be varied along the axial length of the sleeve 422 to elicit different flexibility properties of the sleeve 422. At the proximal end 426b, for example, the filaments 1102 per square inch could be increased as compared to the distal end 426a, thereby resulting in a stiffer or less compressible proximal end 426b.

In some embodiments, the filaments 1102 may be oriented to create anisotropic properties in the sleeve 422 to allow the sleeve 422 to exhibit different mechanical properties in the axial and radial directions.

FIG. 12 is cross-sectional side view of another example of the sleeve 422 of FIG. 4, according to one or more embodiments. Similar to the embodiment of FIG. 10A, the sleeve 422 in FIG. 12 may be made of a plurality of materials. More specifically, the sleeve 422 may include a tip 1202, a base 1204, and an external sleeve 1206. The tip 1202 may be made of a flexible material, such as silicone, that allows the tip 1202 to flex as the jaw members 210, 212 (FIG. 4) move (e.g., open and close). The base 1204 may be made of a more rigid material, such as polyether ether ketone (PEEK). The tip 1202 and the base 1204 may be adjoined at a joint 1208, which may comprise a butt joint or a lap joint, as generally described herein. The external sleeve 1206 may be secured over portions of the tip 1202 and the base 1204 and otherwise extend across the joint 1208 on the outer radial surface.

The multipart sleeve 422 of FIG. 12 may prove advantageous in addressing conflicting design constraints of friction, stiff proximal end 426b, and a flexible distal end 426a. In some embodiments, the external sleeve 1206 may be made of polytetrafluoroethylene (PTFE) and secured to the exterior of the tip 1202 and the base 1204 via a heat shrinking process. In at least one embodiment, the exterior sleeve 1206 may extend across the joint 1208 to secure the tip 1202 to the base 1204. In one or more embodiments, the exterior sleeve 1206 may have or otherwise include an inner layer made of fluorinated ethylene propylene (FEP), which engages the outer radial surfaces of the adjacent portions of the tip 1202 and the base 1204.

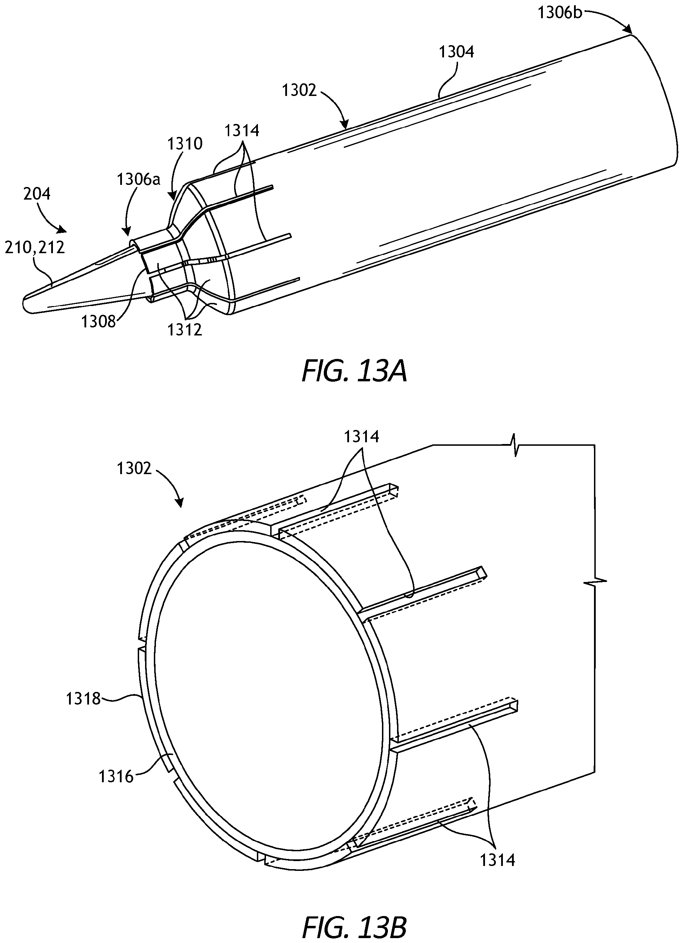

FIG. 13A is an isometric view of the distal end of another embodiment of the surgical tool 200 of FIG. 2. More specifically, FIG. 13A depicts a protective sleeve 1302 that may be similar in some respects to the sleeve 422 of FIG. 4. Similar to the sleeve 422, for example, the sleeve 1302 may be configured to insulate various live (energized) portions of the end effector 204 and/or the wrist 206 (FIG. 4), and thereby protect the patient from stray electrical discharge during operation. As illustrated, the sleeve 1302 may comprise an elongate and generally cylindrical body 1304 having a first or distal end 1306a and a second or proximal end 1306b opposite the distal end 1306a. When the sleeve 1302 is properly positioned for use, the jaw members 210, 212 protrude out an aperture 1308 defined in the distal end 1306a of the body 1304.

As illustrated, the body 1304 may provide a "reformed" distal end 1306a. More specifically, the body 1304 may define a reduced-diameter portion 1310, which provides a transition between the larger-diameter body 1304 and the smaller diameter distal end 1306a.

The sleeve 1302 may provide or otherwise define a plurality of longitudinally-extending fingers 1312 separated by a corresponding plurality of slots 1314. In some embodiments, the fingers 1312 may extend axially through or otherwise be defined at least partially by the reduced-diameter portion 1310 leading to the distal end 1306a. The fingers 1312 may prove advantageous in allowing the jaw members 210, 212 to articulate without risking tearing of the sleeve 1302 at the distal end 1306a. Instead, as the jaw members 210, 212 open and close, the fingers 1310 are able to flex radially outward to accommodate such movement.

FIG. 13B is a cutaway end view of the sleeve 1302 of FIG. 13A, according to one or more embodiments. In some embodiments, as illustrated, the sleeve 1302 may be made of a plurality of layers, shown as a first or "inner" layer 1316 and a second or "outer" layer 1318. In some embodiments, the inner layer 1316 may be made of a material that is more flexible than the material of the outer layer 1318. In such embodiments, for example, the inner layer 1316 may comprise a thermoplastic polyurethane and the outer layer 1318 may comprise a material that is more stiff, such as fluorinated ethylene propylene (FEP) or a perfluoroalkoxy alkane (PFA, or generally any fluoropolymer). The sleeve 1302 may be manufactured as a dual extrusion or alternatively as a two-piece bonded extrusion.

In some embodiments, the inner layer 1316 may not extend into the reduced-diameter portion 1310 (FIG. 13A) but may otherwise terminate prior to the transition to the smaller diameter distal end 1306a (FIG. 13A). Moreover, in such embodiments, the slots 1314 may not be defined in the inner layer 1316, but only in the outer layer 1318. This may prove advantageous in maintaining the more flexible inner layer 1316 material away from the energized (hot) jaw members 210, 212 (FIG. 13A) during operation. Rather, the more rigid outer layer 1318 may be in contact with the energized (hot) jaw members 210,212.

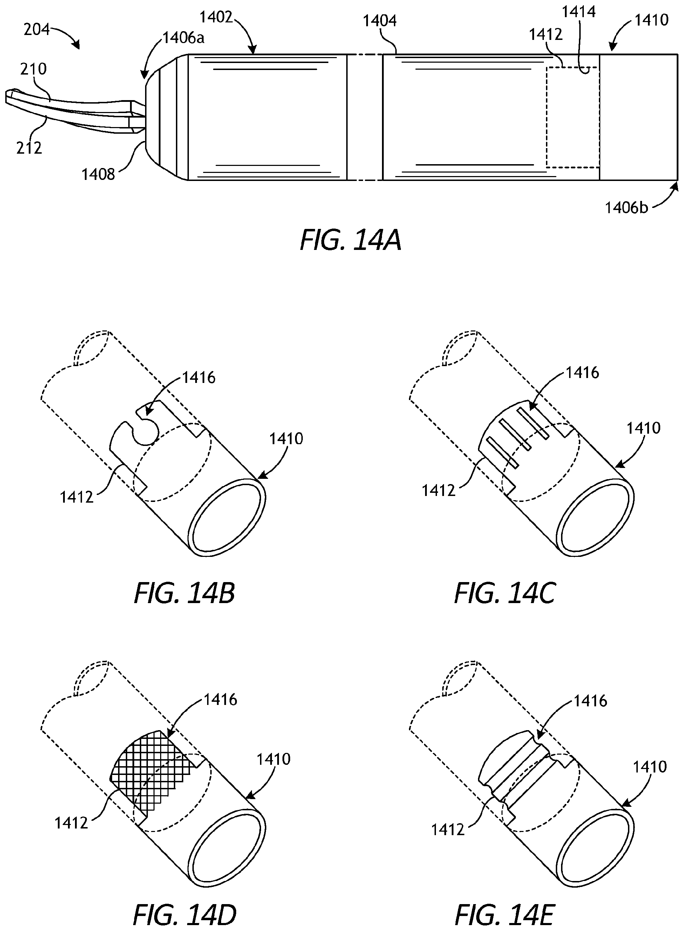

FIG. 14A is an enlarged side view of the distal end of another embodiment of the surgical tool 200 of FIG. 2. More specifically, FIG. 14A depicts the end effector 204 and a protective sleeve 1402 that may be similar in some respects to the sleeve 422 of FIG. 4 and mounted to the end effector 204. Similar to the sleeve 422, for example, the sleeve 1402 may be configured to insulate various live (energized) portions of the end effector 204 and/or the wrist 206 (FIG. 4), and thereby protect the patient from stray electrical discharge during operation. As illustrated, the sleeve 1402 may comprise an elongate and generally cylindrical body 1404 having a first or distal end 1406a and a second or proximal end 1406b opposite the distal end 1406a. When the sleeve 1402 is properly positioned for use, the jaw members 210, 212 protrude out an aperture 1408 defined in the distal end 1406a of the body 1404.

In the illustrated embodiment, the sleeve 1402 may define or otherwise provide a rigid base 1410 at the proximal end 1406b. The rigid base 1410 may form part of the sleeve 1402 and operate to help strengthen the proximal end 1406b for purposes of insertion, attachment, and retention of the sleeve 1402. In at least one embodiment, for instance, the rigid base 1410 may prove advantageous in decreasing friction during assembly of the sleeve 1402 onto the end effector 204.

The body 1404 of the sleeve 1402 may be made of a flexible material, such as silicone or a thermoplastic polyurethane (TPU). In contrast, the rigid base 1410 may be made of a stiffer material, such as, but not limited to, polyether ether ketone (PEEK) or another thermoplastic.

The rigid base 1410 may be coupled or secured to the sleeve 1402 via a variety of attachment means. In at least one embodiment, as illustrated, the rigid base 1410 may be coupled to the sleeve 1402 via a male-female engagement where the rigid base 1410 provides a male end 1412 configured to mate with a female end 1414 of the sleeve 1402. The male-female engagement may comprise a variety of different configurations, without departing from the scope of the disclosure.

FIGS. 14B-14E depict partial isometric views of various examples of the rigid base 1410 being coupled to the sleeve 1402, according to one or more embodiments. In some embodiments, the more flexible material of the sleeve 1402 may be overmolded onto the rigid base 1410, which may define one or more retention features 1416 on the male end 1410 that help maintain the overmolded component in place. More specifically, the retention feature(s) 1416 may provide variations and/or voids in the male end 1410 that allow the material of the sleeve 1402 to creep and otherwise flow into the retention feature(s) 1416 during overmolding. This may result in a stronger bond between the sleeve 1402 and the rigid base 1410.

In FIG. 14B, for example, the retention feature(s) 1416 comprise one or more attachment keys defined on the male end 1410. In FIG. 14C, the retention feature(s) 1416 comprise one or more longitudinal grooves defined on the male end 1410. In FIG. 14D, the retention feature(s) 1416 comprise a knurled finish defined on the male end 1410. Lastly, in FIG. 14E, the retention feature(s) 1416 comprise one or more annular grooves defined on the male end 1410.

The stiffer rigid base 1410 may also prove advantageous in being able to place (position) the sleeve 1404 via a more deterministic locating scheme when coupling the sleeve 1404 to the shaft adapter 400 of FIG. 4 (or alternatively the shaft 202 of FIG. 2 when the shaft adapter 400 is not used). More specifically, the interface on the proximal end of the rigid base 1410 may incorporate, provide, or otherwise define a variety of different attachment features capable of securing the sleeve 1404 in the assembled position and thereby helping to prevent the sleeve 1404 from migrating to the migrated position.

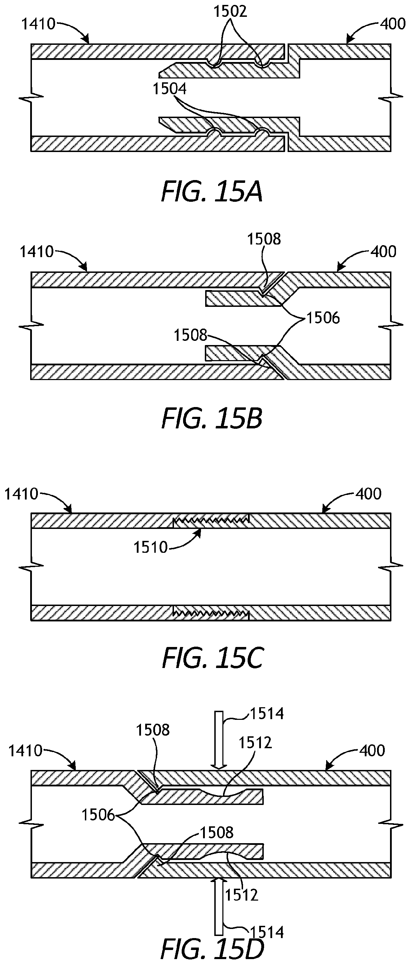

FIGS. 15A-15D are cross-sectional side views of example embodiments of coupling the rigid base 1410 to the shaft adapter 400, according to several embodiments. In FIG. 15A, the shaft adapter 400 may provide or otherwise define one or more annular grooves 1502 (two shown) on its outer surface, and the proximal end of the rigid base 1410 may provide or otherwise define a corresponding one or more annular protrusions 1504 (two shown) configured to mate with the annular grooves 1502 in a snap-fit engagement. The sleeve 1404 (FIG. 14A) may be advanced proximally until the protrusions 1504 locate and snap into engagement with the grooves 1502, which provides a positive indicator that the sleeve 1404 is in the assembled position. The snap-fit engagement between the protrusions 1504 and the grooves 1502 also helps maintain the sleeve 1404 in the assembled position.

In FIG. 15B, the shaft adapter 400 provide or otherwise defines one or more detents 1506 (two shown) on its outer surface, and the proximal end of the rigid base 1410 provides or otherwise defines a corresponding one or more teeth 1508 (two shown) configured to mate with the detents 1506 in a snap-fit engagement. The sleeve 1404 (FIG. 14A) may be advanced proximally until the teeth 1508 snap into engagement with the detents 1506, which provides a positive indicator that the sleeve 1404 is in the assembled position and helps maintain the sleeve 1404 in the assembled position.

In FIG. 15C, the rigid base 1410 may be threaded to the shaft adapter 400 at a threaded interface 1510 which provides a positive indicator that the sleeve 1404 is in the assembled position and simultaneously helps maintain the sleeve 1404 in the assembled position.

In FIG. 15D, the rigid base 1410 may be coupled to the shaft adapter 400 via a detachable pinch-type attachment. More specifically, the rigid base 1410 may provide the detents 1506 and the shaft adapter 400 may provide the corresponding teeth 1508 (although the reverse configuration may be employed also). Moreover, the rigid base 1410 may provide or define one or more radial depressions 1512 (two shown) that provide a location where opposing radial loads 1514 (pinch) may be applied to the outer circumference of the shaft adapter 400. Upon assuming the radial loads 1514, the shaft adapter 400 flexes radially inward into the radial depressions 1512, and a fulcrum effect on the shaft adapter 400 will raise up and release the teeth 1508 from the detents 1506, which serves to release the rigid base 1410 from the shaft adapter 400.

While the foregoing embodiments of FIGS. 15A-15D depict the rigid base 1410 being coupled to the shaft adapter 400, it will be appreciated that the rigid base 1410 may alternatively be coupled to the shaft 202 (FIG. 2) via similar engagement means, without departing from the scope of the disclosure.

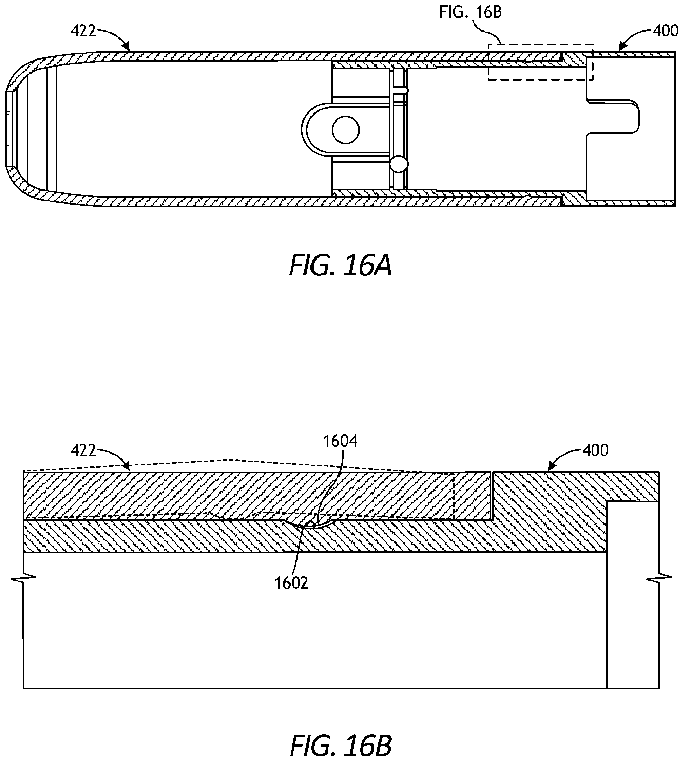

FIG. 16A is an enlarged cross-sectional side view of the shaft adapter 400 and the sleeve 422 of FIG. 4, according to one or more embodiments. More specifically, FIG. 16A depicts the sleeve 422 in the assembled position. In the illustrated embodiment, the sleeve 422 and the shaft adapter 400 may individually or cooperatively help ensure that the sleeve 422 is properly positioned in the assembled position and otherwise provide a positive indicator when the sleeve 422 has moved (migrated) from the assembled position.

FIG. 16B is an enlarged view of the portion of FIG. 16A indicated by the dashed box. As illustrated, the shaft adapter 400 may provide or otherwise define an indentation 1602 and the sleeve 422 may provide or otherwise define a protrusion 1604 configured to mate with the indentation 1602 when the sleeve 422 is in the assembled position. In some embodiments, the indentation 1602 may comprise an annular groove that extends about the entire outer circumference of the shaft adapter 400. In other embodiments, however, the indentation 1602 may comprise one or more discrete depressions formed in the outer radial surface of the shaft adapter 400. Similarly, in some embodiments, the protrusion 1604 may comprise an annular ring or feature that extends about the entire inner radial surface of the sleeve 422. In other embodiments, however, the protrusion 1604 may comprise one or more discrete protruding features formed in the inner radial surface of the sleeve 422 and configured to align with one or more discrete depressions formed in the outer radial surface of the shaft adapter 400.

When the protrusion 1604 is received within the indentation 1602, that may provide a positive indicator that the sleeve 422 is properly positioned on the shaft adapter 400 and otherwise in the assembled position. More specifically, when the protrusion 1604 is properly received within the indentation 1602, the sleeve 422 will exhibit a first diameter that enables the sleeve 422 to pass through a trocar (not shown) that introduces the surgical tool into a patient cavity. When the sleeve 422 begins to move (creep) toward the migrated position, however, the protrusion 1604 will be forced out of the indentation 1602, as indicated in dashed lines. When the protrusion 1604 exits the indentation 1602, the diameter of the sleeve 422 increases at that location to a second diameter greater that the first diameter. The enlarged second diameter will prevent the sleeve 422 from traversing the trocar without binding against the inner wall of the trocar. Consequently, if the sleeve 422 binds against the interior of the trocar, that may be a positive indicator that the sleeve 422 is not in the assembled position and should be resituated to the proper position.

As will be appreciated, the orientation of the indentation(s) 1602 and the protrusion(s) 1604 may be reversed, where the indentation(s) 1602 is defined on the sleeve 422 and the protrusion(s) 1604 is defined on the shaft adapter 400, without departing from the scope of the disclosure.

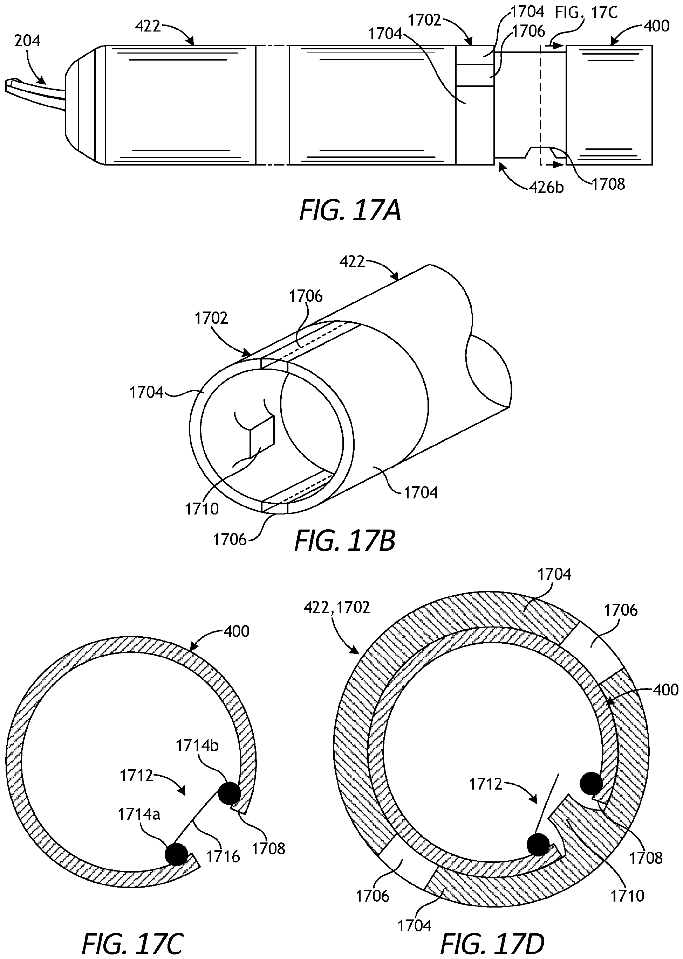

FIG. 17A is an enlarged cross-sectional side view of the shaft adapter 400 and the sleeve 422 of FIG. 4, according to one or more additional embodiments. More specifically, FIG. 17A depicts the sleeve 422 in the migrated position and otherwise offset from the assembled position. In the illustrated embodiment, an expandable ring 1702 may be provided or otherwise defined at the proximal end 426b of the sleeve 422. The expandable ring 1702 may include one or more arcuate portions 1704 made of a hard material and separated by a spacer 1706 made of a flexible material. Moreover, the shaft adapter 400 may define a window 1708 configured to interact with the expandable ring 1702.

FIG. 17B is an enlarged isometric end view of the expandable ring 1702, according to one or more embodiments. The arcuate portions 1704 of the expandable ring 1702 may be made of a hard material such as, but not limited to, polyether ether ketone (PEEK) or another thermoplastic. While two arcuate portions 1704 are depicted in FIG. 17B, it will be appreciated that more than two may be employed, without departing from the scope of the disclosure. The spacers 1706 (two shown) that angularly interpose the arcuate portions 1704 may be made of a flexible material such as, but not limited to, silicone, rubber, or an elastomer.

As illustrated, the expandable ring 1702 may further define or otherwise provide a boss 1710 configured to align with and be received within the window 1708 (FIG. 17A) of the shaft adapter 400 (FIG. 17A). The boss 1710 may comprise a radial protrusion that extends radially inward from the inner wall of the sleeve 422 or otherwise from the inner wall of the expandable ring 1702. Because the boss 1710 extends radially inward, the expandable ring 1702 will have to radially expand as the sleeve 422 is received over the outer circumference of the shaft adapter 400 and advanced toward the assembled position. The flexible spacer(s) 1706 allow the expandable ring 1702 to expand radially outward to accomplish this. The sleeve 422 is advanced proximally over the shaft adapter 400 until the boss 1710 locates and is received within the window 1708.

FIG. 17C is a cross-sectional end view of the shaft adapter 400 of FIG. 17A as taken along the indicated line of FIG. 17A. More particularly, FIG. 17C depicts the interior of the shaft adapter 400 at the window 1708. As illustrated, the shaft adapter 400 may include a shorting switch 1712 positioned within the interior of the shaft adapter 400 and accessible via the window 1708. The shorting switch 1712 includes a hot contact 1714a, a shorting contact 1714b, and a shorting wire 1716 extendable between the hot and shorting contacts 1714a,b. When the shorting wire 1716 extends between the hot and shorting contacts 1714a,b, the electrical circuit providing electrical energy to the end effector 204 (FIG. 17A) is shorted, thus rendering the end effector 204 without electrical energy. In contrast, when the shorting wire 1716 is moved out of contact between the hot and shorting contacts 1714a,b, the electrical energy can flow to the end effector 204.

FIG. 17D is a cross-sectional end view of the shaft adapter 400 encircled by the sleeve 422 of FIG. 17A. More particularly, FIG. 17D depicts the expandable ring 1702 surrounding the shaft adapter 400 and the boss 1710 received within the window 1708. When the boss 1710 is received within the window 1708, the boss 1710 engages the shorting wire 1716 and disrupts the shorting switch 1712, which allows electrical energy to be supplied to the end effector 204 (FIG. 17A). Accordingly, the end effector 204 cannot be energized until the boss 1710 is properly received within the window 1708, which does not occur until the sleeve 422 is properly positioned in the assembled position.

Consequently, the expandable ring 1702 may provide a positive indicator that the sleeve 422 is properly placed in the assembled position. Alternatively, the expandable ring 1702 may also provide a positive indicator that the sleeve 422 has moved (migrated) from the assembled position to the migrated position. More specifically, when the sleeve 422 moves to the migrated position and the boss 1708 exits the window 1708, the shorting switch 1712 is able to short the electrical circuit once again, which shuts off power to the end effector 204 (FIG. 17A) and thereby provides a positive indicator that the sleeve 422 has migrated.

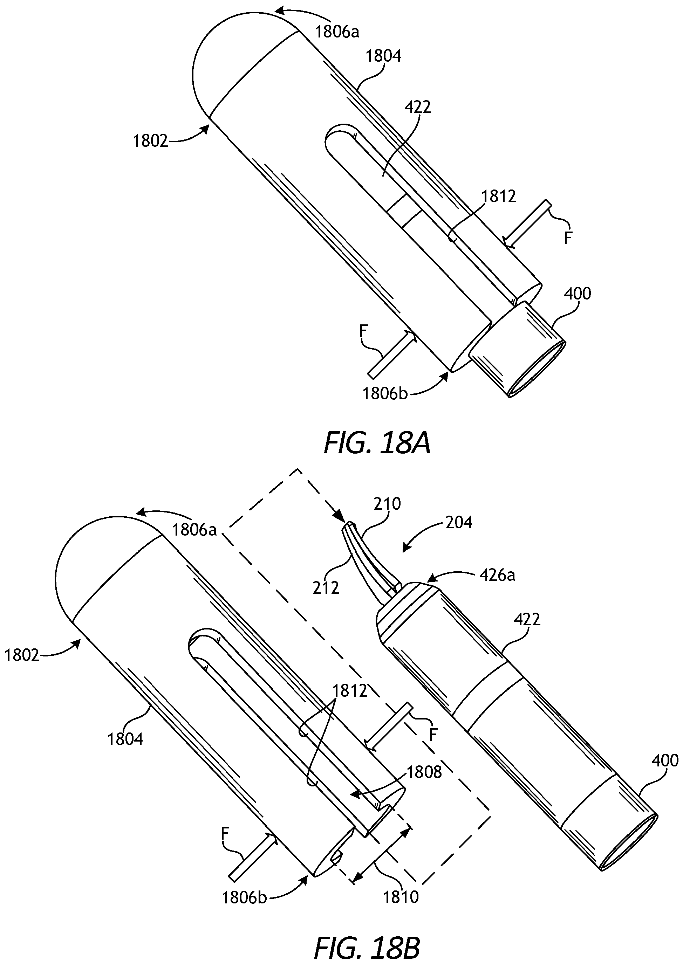

FIGS. 18A and 18B are isometric assembled and exploded views, respectively, of an example sleeve insertion tool 1802 used in conjunction with the end effector 204, accordingly to one or more embodiments. The sleeve insertion tool 1802 may be used to help install (assemble) the sleeve 422 on the end effector 204 in the assembled position and simultaneously protect a user (e.g., a scrub nurse, surgeon, etc.) from inadvertent injury caused by accidental contact with the jaw members 210, 212. As will be appreciated, the jaw members 210, 212 are required to be exceptionally sharp and the user is commonly tasked with assembling the sleeve 422 over the jaw members 210, 212. If proper precaution is not taken, the user may inadvertently cut or puncture his/her hand(s) by coming into contact with the jaw members 210, 212. The sleeve insertion tool 1802 may prove advantageous in mitigating the occurrence of cuts or punctures caused accidental mishandling of the end effector 204.

The sleeve insertion tool 1802 may be designed to temporarily occlude (cover) the distal end of the end effector 204 and, more particularly, the exposed jaw members 210, 212 protruding from the distal end 426a of the sleeve 422. As illustrated, the sleeve insertion tool 1802 includes an elongate, generally cylindrical body 1804 having a closed distal end 1806a and an open proximal end 1806b opposite the distal end 1806a. The body 1804 may be made of a variety of materials including, but not limited to, plastic, metal, rubber, an elastomer, silicone, and any combination thereof.

The body 1804 defines an inner chamber 1808 that exhibits an inner diameter 1810 large enough to be extended over and otherwise receive the end effector 204 and the sleeve 422. In some embodiments, the body 1804 may define one or more longitudinal slots 1812 (two shown) that extend from the proximal end 1806b toward the distal end 1806a. The slots 1812 create weak points in the body 1804 that allow a user to pinch and thereby collapse the body 1804 against the outer radial surface of the sleeve 422 during installation. This allows the user to advance the sleeve 422 toward the assembled position by gripping and moving the sleeve insertion tool 1802 instead of directly contacting the outer surface of the sleeve 422.

While two slots 1812 are shown in the illustrated embodiments, more or less than two slots 1812 may alternatively be employed, without departing from the scope of the disclosure. In some embodiments, as illustrated, one or more of the slots 1812 may exhibit an axial length that is greater than half the overall length of the body 1804. In other embodiments, the axial length of the slots 1812 may be less than half the overall length of the body 1804, without departing from the scope of the disclosure.

Example assembly of the sleeve 422 with the assistance of the sleeve insertion tool 1802 is now provided. The sleeve insertion tool 1802 and the sleeve 422 may be packaged in a common sterile packaging and shipped together. Upon opening the sterile pack, a user (e.g., a scrub nurse, surgeon, etc.) may extend the sleeve 422 partially onto the end effector 204. In other embodiments, however, the sleeve 422 may be pre-assembled into the sleeve insertion tool 1802 or the user may be required to manually insert the sleeve 422 into the inner chamber 1808 of the sleeve insertion tool 1802. The sleeve insertion tool 1802 may then be used to advance the sleeve 422 to the assembled position.

The proximal end 1806b of the sleeve insertion tool 1802 may be extended over the end effector 204 such that the jaw members 210, 212 are received into the inner chamber 1808, thus protecting the user from being cut by the jaw members 210, 212. The user may then advance the sleeve insertion tool 1802 proximally relative to the end effector 204 and the shaft adapter 400 (or alternatively the shaft 202 of FIG. 2) and simultaneously advance the sleeve 422 to the assembled position.

In some embodiments, sleeve 422 may be advanced to the assembled position by first applying an opposing radial load F on the sleeve insertion tool 1802 at or near the proximal end 1806b, such as by pinching the sleeve insertion tool 1802 with the thumb and index fingers of one hand. The radial load F may cause the inner radial surface of the sleeve insertion tool 1802 to engage and otherwise grip the outer radial surface of the sleeve 422. In some embodiments, the inner radial surface at or near the proximal end 1806b may include a gripping interface, such as a knurled surface or a ribbed contour, configured to help grip the outer radial surface of the sleeve 422. The slots 1812 allow the body 1804 to flex radially inward, and the sleeve insertion tool 1802 may then be advanced proximally relative to the end effector 204 without potentially binding (crumpling) the sleeve 422 within the insertion tool.

Once the sleeve 422 reaches the assembled position, the sleeve insertion tool 1802 may be retracted distally to thereby remove the sleeve insertion tool 1802 from the end effector 204 and leave the sleeve 422 in place. In embodiments where the sleeve insertion tool 1802 is made of a pliable material (e.g., an elastomer or silicone), the slots 1812 may allow the sleeve insertion tool 1802 to be "peeled away" from the end effector 204 after assembly. In such embodiments, the user may grasp the body 1804 at or near the proximal end 1806b and the slots 1812 may help progressively detach the body 1804 similar to how a banana is peeled.

In some embodiments, the sleeve insertion tool 1802 may also be used to help remove the sleeve 422. In such embodiments, the sleeve insertion tool 1802 may be reinstalled over the sleeve 422, and the radial load F may again be applied to engage and otherwise grip the outer radial surface of the sleeve 422. Once the sleeve 422 is engaged, the user may move the sleeve insertion tool 1802 and the sleeve 422 distally together. In such embodiments, the gripping interface mentioned above may prove advantageous to help grip the outer radial surface of the sleeve 422.

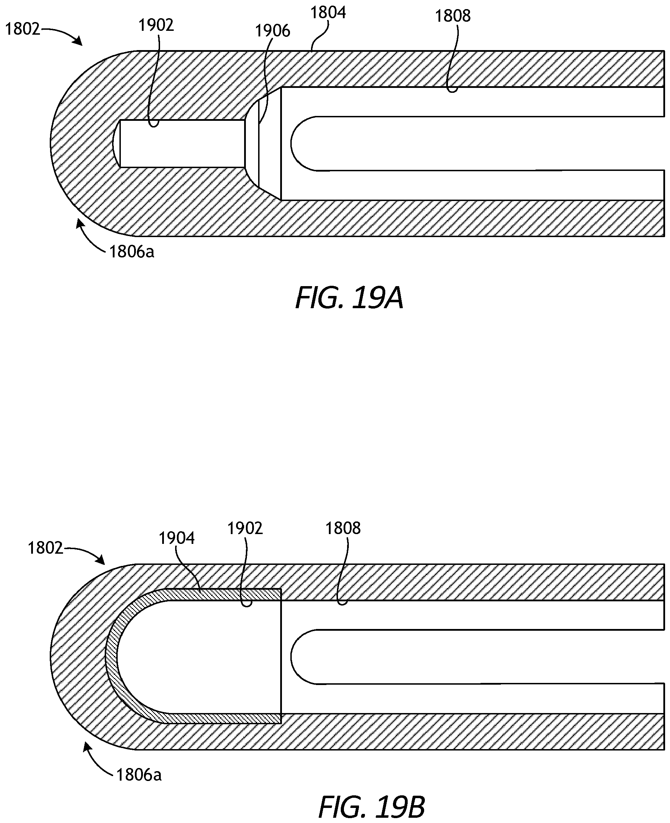

FIG. 19A is a cross-sectional side view of one example of the sleeve insertion tool 1802, according to one or more embodiments. As illustrated, the body 1804 of the sleeve insertion tool 1802 may provide or otherwise define a jaw cavity 1902 at or near the distal end 1806a. In some embodiments, the jaw cavity 1902 (alternately referred to as a "relief pocket") may form an integral extension of the inner chamber 1808. In other embodiments, however, the jaw cavity 1902 may comprise a separate compartment and extend distally from the inner chamber 1808. The jaw cavity 1902 may be sized and otherwise configured to receive the jaw members 210, 212 (FIG. 18B) therein when the sleeve insertion tool 1802 is extended over the end effector 204 (FIG. 18B). In the illustrated embodiment, the jaw cavity 1902 extends substantially coaxial with the inner chamber 1808. In other embodiments, however, the jaw cavity 1902 may extend at an angle offset from the centerline of the body 1804 to accommodate the angled contour of the jaw members 210, 212.

In some embodiments, a sleeve stop 1906 may be provided or otherwise defined within the inner chamber 1808 of the sleeve insertion tool 1802. The sleeve stop 1906 may provide a transition surface between the inner chamber 1808 and the jaw cavity 1902. The sleeve stop 1906 may be configured to receive the distal end 426a (FIG. 18B) of the sleeve 422 (FIGS. 18A-18B) when the sleeve 422 is introduced into the inner chamber 1808. In some embodiments, as illustrated, the sleeve stop 1906 may exhibit an arcuate conical cross-section configured to mate with the corresponding arcuate conical exterior profile of the distal end 426a of the sleeve 422. In other embodiments, however, the sleeve stop 1906 may exhibit any other cross-sectional shape, without departing from the scope of the disclosure.

FIG. 19B is a cross-sectional side view of another example of the sleeve insertion tool 1802, according to one or more additional embodiments. As illustrated, the jaw cavity 1902 is again provided at or near the distal end 1806a of the sleeve insertion tool 1802. In the illustrated embodiment, however, a cap 1904 may be positioned within the jaw cavity 1902. In some embodiments, as illustrated, the cap 1904 may comprise a generally dome-shaped or hemispherical structure. In other embodiments, however, the cap 1904 may exhibit a polygonal cross-section, without departing from the scope of the disclosure.

The cap 1904 may be configured to prevent the jaw members 210, 212 (FIG. 18B) from piercing the distal end 1806a of the sleeve insertion tool 1802 when installing the sleeve 422 (FIGS. 18A- 18B). To accomplish this, the cap 1904 may be made of a variety of rigid materials such as, but not limited to, a metal, a plastic (e.g., acrylonitrile butadiene styrene, polycarbonate, polyether ether ketone, etc.), a composite material, and any combination thereof. In some embodiments, the cap 1904 may be secured within the jaw cavity 1902 using an adhesive or via sonic welding. In other embodiments, however, the sleeve insertion tool 1802 may be overmolded onto the cap 1904, without departing from the scope of the disclosure.

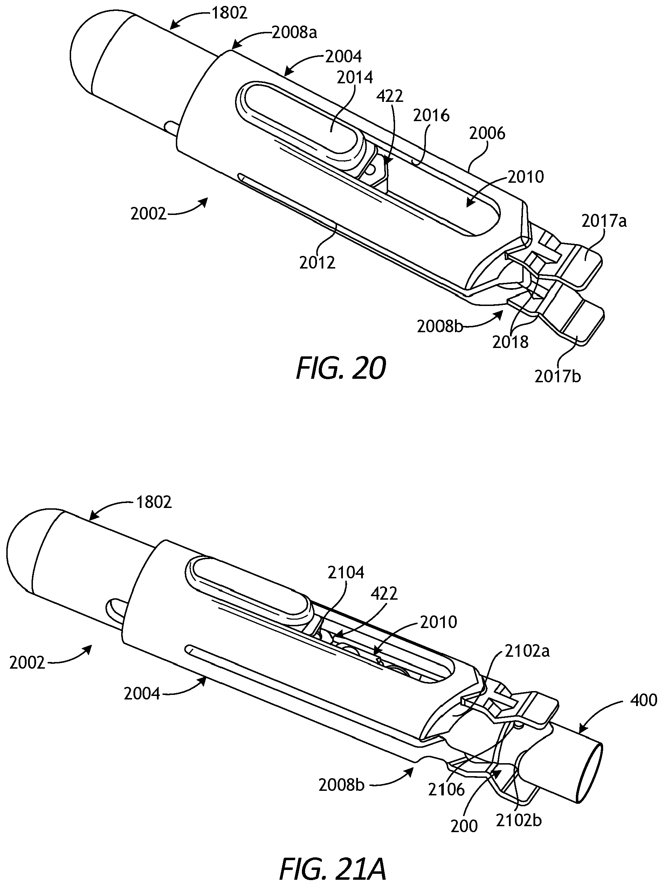

FIG. 20 is an isometric view of an example sleeve install assembly 2002, according to one or more embodiments. The sleeve install assembly 2002 (hereafter "the assembly 2002") may be used to install the sleeve 422 (partially occluded) on the distal end of the surgical tool 200 of FIGS. 2 and 4.

As illustrated, the assembly 2002 may include the sleeve insertion tool 1802 and a locator 2004. The locator 2004 may comprise an elongate, generally cylindrical body 2006 having a first or distal end 2008a, a second or proximal end 2008b, and an interior 2010 that extends between the distal and proximal ends 2008a,b. The body 2006 may be generally open at both ends 2008a,b and may define one or more longitudinal slots 2012 (one shown) extending from the proximal end 2008b toward the distal end 2008a. The slots 2012 allow the locator 2004 to open up (e.g., a clamshell) to receive the sleeve insertion tool 1802 within the interior 2010 via the proximal end 2008b.

In the illustrated embodiment, the sleeve insertion tool 1802 includes at least one guide rib 2014 defined on the outer radial surface of the body 1804. The guide rib 2014 may be configured to extend radially through a corresponding channel 2016 defined by the locator 2004. As illustrated, the channel 2016 extends longitudinally, and the guide rib 2014 may be configured to translate (slide) axially within the channel 2016 during operation of the assembly 2002. In some embodiments, the guide rib 2014 may be engageable by a user (e.g., a user's thumb or index finger) to manipulate the axial position of the sleeve insertion tool 1802.

In some embodiments, the locator 2004 may provide a release mechanism at or near the distal end 2008b. The release mechanism may be configured to help release and otherwise remove the assembly 2002 from a surgical tool after properly placing the sleeve 422 in the assembled position. In the illustrated embodiment, the release mechanism comprises a pair of opposing tabs 2017a and 2016b. The tabs 2017a,b may provide or otherwise define opposing fulcrum points 2018 used to leverage portions of the body 2006 radially outward and out of axial engagement with the sleeve 422 once placed in the assembled position.

FIGS. 21A-21C are progressive isometric views of the assembly 2002 in the process of installing the sleeve 422 on the distal end of the surgical tool 200. The assembly 2002 may be used to place (install) the sleeve 422 in the assembled position relative to the shaft adapter 400, or the distal end of the shaft 202 of FIG. 2 in embodiments that omit the shaft adapter 400.

Referring first to FIG. 21A, the sleeve insertion tool 1802 is received or positioned within the interior 2010 of the locator 2004, and the sleeve 422 may be preloaded within the sleeve insertion tool 1802, as generally described above. The assembly 2002 may then receive the distal end of the surgical tool 200 by introducing the surgical tool 200 into the interior 2010 of the locator 2004 via the open proximal end 2008b. The assembly 2002 may be advanced proximally until the distal end of the surgical tool 200 (e.g., the end effector 204 of FIGS. 2 and 4) also extends into the sleeve insertion tool 1802.

In some embodiments, the locator 2004 may be configured to rotationally (angularly) align the sleeve 422 relative to the distal end of the surgical tool 200. To accomplish this, the assembly 2002 may include an alignment feature. In the illustrated embodiment, the alignment feature may comprise a first profile 2102a provided by the locator 2004 and configured to mate with a corresponding second profile 2102b defined on the shaft adapter 400 (or alternatively the end of the shaft 202 of FIG. 2). In the illustrated embodiment, the profiles 2102a,b provide mating V-shaped angled surfaces. In other embodiments, however, the profiles 2102a,b may exhibit other profile shapes or configurations, without departing from the scope of the disclosure. As the assembly 2002 is advanced proximally relative to the surgical tool 200, the opposing profiles 2102a,b will eventually come into contact with each other and slidingly engage to urge the assembly 2002 to rotate into a predetermined angular alignment.

In some embodiments, the sleeve 422 may be configured to be secured to the shaft adapter 400 (or alternatively the end of the shaft 202 of FIG. 2) upon being moved to the assembled position. More specifically, as illustrated, the sleeve 422 may define one or more apertures 2104 at or near its proximal end and the shaft adapter 400 may provide or define one or more posts 2106 sized to be received by and otherwise mate with the aperture 2104. As the sleeve 422 is moved to the assembled position, the post 2106 will locate and be received within the aperture 2104. The coupling engagement between the aperture 2104 and the post 2106 may prove advantageous in helping maintain the sleeve 422 in the assembled position during operation.

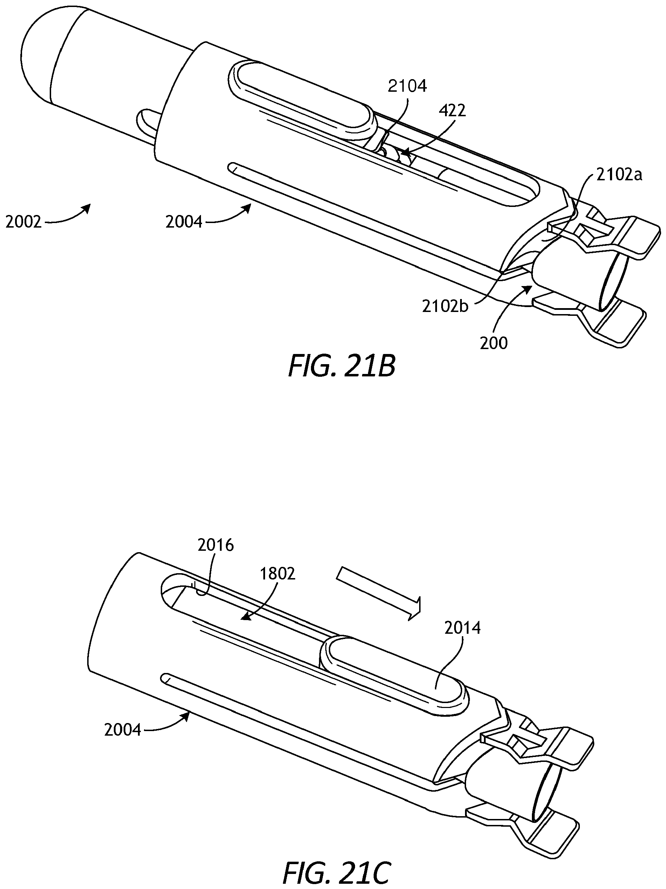

In FIG. 21B, the assembly 2002 has moved proximally relative to the distal end of the surgical tool 200 until the profile 2102a of the locator 2004 slidingly engages and bottoms out in the profile 2102b of the shaft adapter 400 (or alternatively the end of the shaft 202 of FIG. 2). The angled surfaces of the profiles 2102 urge the assembly 2002 to rotate to the predetermined angular orientation relative to the surgical tool 200, which simultaneously aligns the post 2106 (FIG. 21A) with the aperture 2104 such that the post 2106 can be received by the aperture 2104 upon moving the sleeve 422 to the assembled position.

In FIG. 21C, the sleeve 422 (FIGS. 21A-21B) is moved to the assembled position by advancing the sleeve insertion tool 1802 proximally relative to the locator 2004. In some embodiments, the sleeve insertion tool 1802 may be advanced proximally by manually engaging the guide rib 2014 with a thumb or index finger and manually urging the sleeve insertion tool 1802 proximally. In other embodiments, however, a user may simply push on the distal end of the sleeve insertion tool 1802. The guide rib 2014 slides within the channel 2016 as the sleeve insertion tool 1802 moves, which helps maintain the sleeve 422 oriented rotationally so that the aperture 2104 (FIGS. 21A-21B) can properly locate the post 2106 (FIG. 21A).

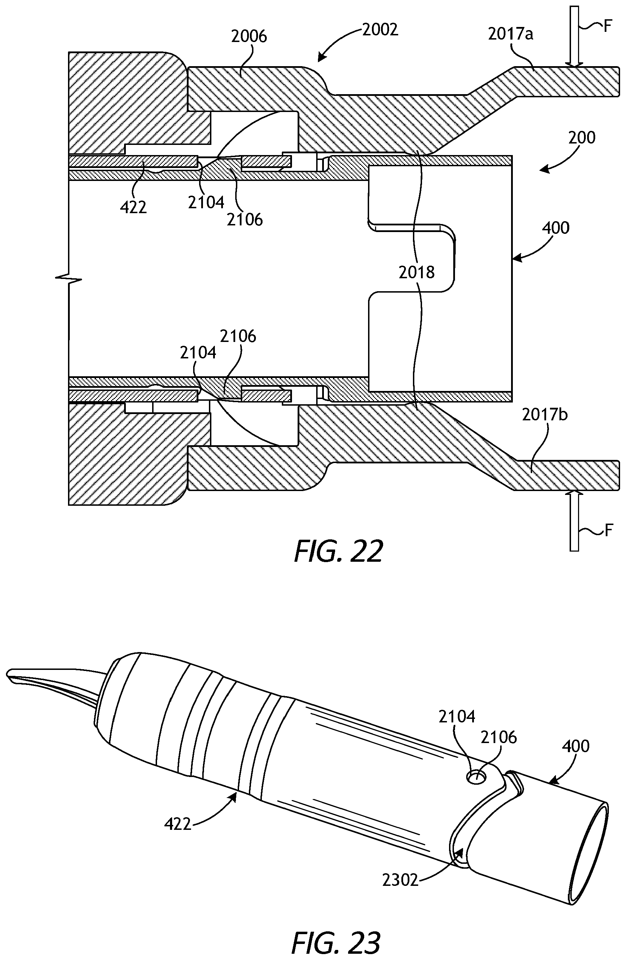

FIG. 22 is an enlarged cross-sectional side view of the assembly 2002 after placing the sleeve 422 in the assembled position relative to the distal end of the surgical tool 200. As illustrated, the sleeve 422 defines two apertures 2104 on angularly opposite sides of the sleeve 422, and the shaft adapter 400 defines two corresponding posts 2106 on angularly opposite sides of shaft adapter 400 and received within the respective apertures 2104.

With the sleeve 422 in the assembled position, the assembly 2002 may be released from the surgical tool 200 by actuating the release mechanism. More specifically, a user (e.g., a scrub nurse or surgeon) may place an opposing radial load F on the tabs 2017a,b, such as by using the thumb and index finger. The opposing radial load F is transmitted to the opposing fulcrum points 2018, which act on the outer radial surface of the shaft adapter 400 and result in portions of the body 2006 expanding radially outward and out of axial alignment (or engagement) with the sleeve 422. This allows the assembly 2002 to be moved distally and removed from the surgical tool 200.

FIG. 23 is an isometric view of the sleeve 422 in the assembled position. As illustrated, the post 2106 is received within the aperture 2104 to help maintain the sleeve 422 in the assembled position. When it is desired to remove the sleeve 422, a tool with a pointed (e.g., somewhat sharp) end may be inserted in a gap 2302 between the sleeve 422 and the shaft adapter 400 (or alternatively the end of the shaft 202 of FIG. 2) and the sleeve 422 may be pried up to disengage the aperture(s) 2104 from the post(s) 2106. The sleeve 422 may thereafter be pulled or peeled off the shaft adapter 400 in the distal direction.

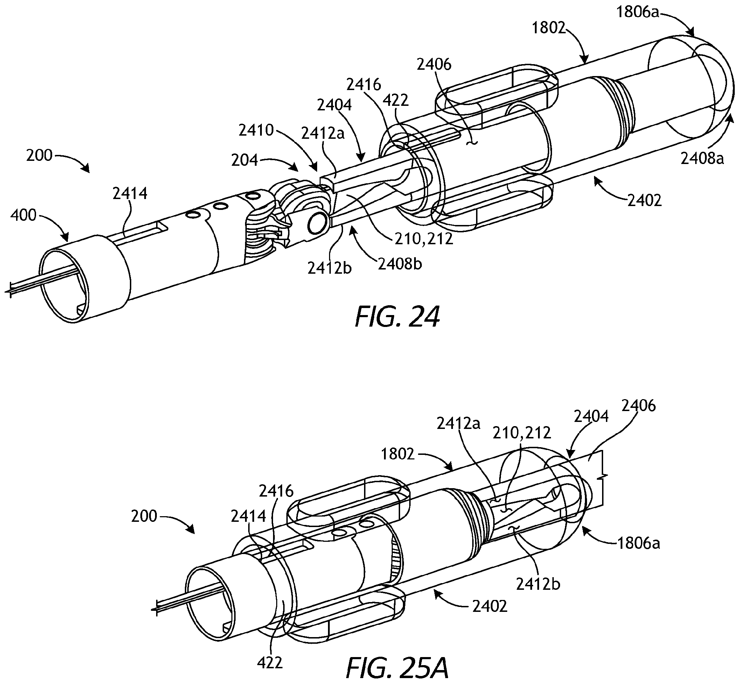

FIG. 24 is an isometric view of another example sleeve install assembly 2402, according to one or more embodiments. The sleeve install assembly 2402 (hereafter "the assembly 2402") may be used to install the sleeve 422 on the distal end of the surgical tool 200.

As illustrated, the assembly 2402 may include the sleeve insertion tool 1802 and an alignment tool 2404. The alignment tool 2404 may comprise an elongate, generally cylindrical shaft 2406 having a first or distal end 2408a and a second or proximal end 2408b opposite the distal end 2408a. In the illustrated embodiment, the distal end 1806a of the sleeve insertion tool 1802 is open to allow the shaft 2406 to extend out the distal end 1806a during operation (actuation) of the assembly 2402. The sleeve 422 may be preloaded within the sleeve insertion tool 1802, as generally described above.

An alignment feature 2410 may be provided at or near the proximal end 2408b of the shaft 2406 and may be used to help angularly align the sleeve 422 to a predetermined angular orientation relative to the shaft adapter 400 (or alternatively the end of the shaft 202 of FIG. 2). More specifically, in the illustrated embodiment the alignment feature 2410 includes opposing alignment jaws 2412a and 2412b configured to receive and seat the opposing jaw members 210, 212 of the end effector 204. In some embodiments, the alignment jaws 2412a,b may define arcuate inner surfaces configured to cradle the jaw members 210, 212.

When the jaw members 210, 212 are properly received between the alignment jaws 2412a,b, an alignment slot 2414 defined on the shaft adapter 400 becomes angularly aligned with a radial tab 2416 defined on the inner radial surface of the sleeve 422. Accordingly, properly receiving the jaw members 210, 212 between the alignment jaws 2412a,b angularly aligns the alignment slot 2414 with the radial tab 2416.

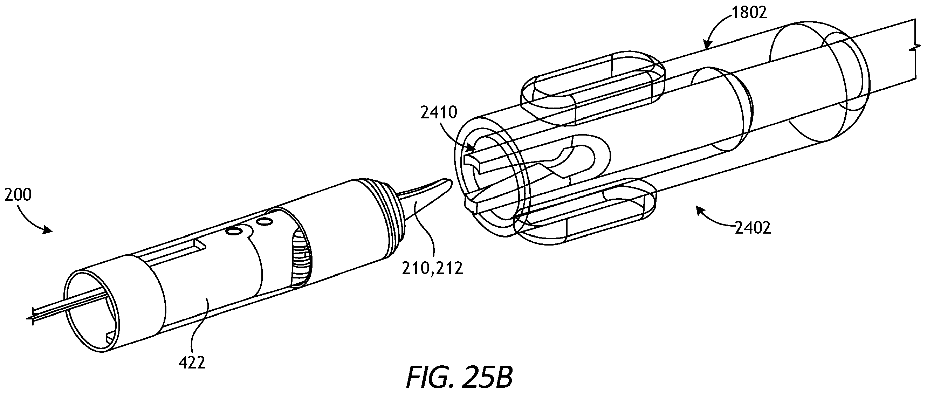

FIGS. 25A and 25B are progressive isometric views of the assembly 2402 in the process of installing the sleeve 422 on the distal end of the surgical tool 200. The assembly 2402 may be used to place (install) the sleeve 422 in the assembled position relative to the shaft adapter 400 or, in embodiments that omit the shaft adapter, the distal end of the shaft 202 of FIG. 2.

Referring first to FIG. 25A, to move the sleeve 422 to the assembled position, the sleeve insertion tool 1802 is moved proximally relative to the surgical tool 200. Since the jaw members 210, 212 are received between the alignment jaws 2412a,b, moving the sleeve insertion tool 1802 proximally will simultaneously move the shaft 2406 of the alignment tool 2404 to telescope out the distal end 1806a of the sleeve insertion tool 1802. Moreover, because the jaw members 210, 212 are received between the alignment jaws 2412a,b, the alignment slot 2414 defined on the shaft adapter 400 will be angularly aligned with the radial tab 2416 on the sleeve 422. Accordingly, advancing the sleeve 422 distally will allow the radial tab 2416 to locate and be received within the alignment slot 2414 and thereby secure the sleeve 422 in the assembled position.

In FIG. 25B, the assembly 2402 is detached (disengaged) from the surgical tool 200 by being moved distally relative to the surgical tool 200. The sleeve 422 remains in the assembled position and the alignment feature 2410 eventually disengages from the jaw members 210, 212. Accordingly, properly positioning the sleeve 422 in the assembled position with the assembly 2402 requires both proximal and distal movement of the sleeve insertion tool 1802.

The following is a non-exhaustive list of embodiments that may or may not be claimed:
A. A sleeve insertion tool that includes an elongate cylindrical body having a closed distal end, an open proximal end, and an inner chamber extending from the proximal end toward the distal end, a jaw cavity defined within the body and extending distally from the inner chamber, and a protective sleeve receivable within the inner chamber and defining an aperture at a distal end thereof, wherein an end effector with one or more jaw members is receivable within the interior and the one or more jaw members are extendable through the aperture of the protective sleeve and receivable into the jaw cavity.
B. A method that includes positioning a sleeve insertion tool proximate a distal end of a surgical tool having an end effector with one or more jaw members, the sleeve insertion tool including an elongate cylindrical body having a closed distal end, an open proximal end, and an inner chamber extending from the proximal end toward the distal end, and a protective sleeve received within the inner chamber. The method further including advancing the sleeve insertion tool over the distal end of the surgical tool whereby the end effector enters the inner chamber and the one or more jaw members protrude through an aperture defined at a distal end of the protective sleeve, receiving the one or more jaw members in a jaw cavity defined within the body and extending distally from the inner chamber, and advancing the sleeve insertion tool proximally relative to the surgical tool and thereby locating the protective sleeve in an assembled position on the surgical tool.

Each of embodiments A and B may have one or more of the following additional elements in any combination: Element 1: wherein the body comprises a material selected from the group consisting of a plastic, a metal, a rubber, an elastomer, silicone, or any combination thereof. Element 2: further comprising one or more longitudinal slots defined in the body and extending from the proximal end toward the distal end. Element 3: further comprising a sleeve stop defined within the inner chamber and providing a transition surface between the inner chamber and the jaw cavity. Element 4: wherein the distal end of the protective sleeve engages the sleeve stop when the protective sleeve is received within the inner chamber. Element 5: wherein the sleeve stop exhibits an arcuate conical cross-section. Element 6: further comprising a cap positioned within the jaw cavity. Element 7: wherein the cap comprises a hemispherical structure. Element 8: wherein the cap comprises a rigid material selected from the group consisting of a metal, a plastic, a composite material, or any combination thereof. Element 9: wherein the body is overmolded onto the cap.

Element 10: wherein advancing the sleeve insertion tool proximally relative to the surgical tool comprises applying an opposing radial load on the sleeve insertion tool at or near the proximal end, and engaging an outer radial surface of the protective sleeve with an inner radial surface of the sleeve insertion tool. Element 11: further comprising retracting the sleeve insertion tool distally relative to the surgical tool, and maintaining the protective sleeve in the assembled position as the sleeve insertion tool retracts distally. Element 12: wherein the sleeve insertion tool further includes one or more longitudinal slots defined in the body and extending from the proximal end toward the distal end, the method further comprising removing the sleeve insertion tool by peeling the sleeve insertion tool off the distal end of the surgical tool along the one or more longitudinal slots. Element 13: wherein the sleeve insertion tool further comprises a sleeve stop defined within the inner chamber, the method further comprising engaging the distal end of the protective sleeve against the sleeve stop when the protective sleeve is introduced into the inner chamber. Element 14: wherein the sleeve insertion tool further comprises a cap positioned within the jaw cavity, the method further comprises engaging the one or more jaw members against the cap while the sleeve insertion tool is advanced proximally relative to the surgical tool. Element 15: wherein the cap comprises a rigid material selected from the group consisting of a metal, a plastic, a composite material, or any combination thereof. Element 16: wherein positioning the sleeve insertion tool proximate the distal end of a surgical tool is preceded by introducing the protective sleeve at least partially into the inner chamber. Element 17: wherein positioning the sleeve insertion tool proximate the distal end of a surgical tool is preceded by receiving the sleeve insertion tool with the protective sleeve pre-assembled within the inner chamber. Element 18: further comprising protecting a user from accidental cuts caused by the one or more jaw members with the sleeve insertion tool.

By way of non-limiting example, exemplary combinations applicable to A and B include: Element 3 with Element 4; Element 3 with Element 5; Element 6 with Element 7; Element 6 with Element 8; Element 6 with Element 9; and Element 14 with Element 15.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of' or "consist of' the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of' preceding a series of items, with the terms "and" or "or" to separate any of the items, modifies the list as a whole, rather than each member of the list (i.e., each item). The phrase "at least one of' allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

## Claims

1. A sleeve insertion tool, comprising:
an elongate cylindrical body having a closed distal end, an open proximal end, and an inner chamber extending from the proximal end toward the distal end;
a jaw cavity defined within the body and extending distally from the inner chamber; and
a protective sleeve receivable within the inner chamber and defining an aperture at a distal end thereof,
wherein an end effector with one or more jaw members is receivable within the interior and the one or more jaw members are extendable through the aperture of the protective sleeve and receivable into the jaw cavity.

2. The sleeve insertion tool of claim 1, wherein the body comprises a material selected from the group consisting of a plastic, a metal, a rubber, an elastomer, silicone, or any combination thereof.

3. The sleeve insertion tool of claim 1 or claim 2, further comprising one or more longitudinal slots defined in the body and extending from the proximal end toward the distal end.

4. The sleeve insertion tool of any preceding claim, further comprising a sleeve stop defined within the inner chamber and providing a transition surface between the inner chamber and the jaw cavity.

5. The sleeve insertion tool of any preceding claim, wherein the distal end of the protective sleeve engages the sleeve stop when the protective sleeve is received within the inner chamber.

6. The sleeve insertion tool of claim 4 or claim 5, wherein the sleeve stop exhibits an arcuate conical cross-section.

7. The sleeve insertion tool of any preceding claim, further comprising a cap positioned within the jaw cavity, wherein the cap preferably comprises a hemispherical structure and/or wherein the body is preferably overmolded onto the cap.

8. A method, comprising:
positioning a sleeve insertion tool proximate a distal end of a surgical tool having an end effector with one or more jaw members, the sleeve insertion tool including:
an elongate cylindrical body having a closed distal end, an open proximal end, and an inner chamber extending from the proximal end toward the distal end; and
a protective sleeve received within the inner chamber;
advancing the sleeve insertion tool over the distal end of the surgical tool whereby the end effector enters the inner chamber and the one or more jaw members protrude through an aperture defined at a distal end of the protective sleeve;
receiving the one or more jaw members in a jaw cavity defined within the body and extending distally from the inner chamber; and
advancing the sleeve insertion tool proximally relative to the surgical tool and thereby locating the protective sleeve in an assembled position on the surgical tool.

9. The method of claim 8, wherein advancing the sleeve insertion tool proximally relative to the surgical tool comprises:
applying an opposing radial load on the sleeve insertion tool at or near the proximal end; and
engaging an outer radial surface of the protective sleeve with an inner radial surface of the sleeve insertion tool.

10. The method of claim 8 or claim 9, further comprising:
retracting the sleeve insertion tool distally relative to the surgical tool; and
maintaining the protective sleeve in the assembled position as the sleeve insertion tool retracts distally.

11. The method of any one of claims 8 to 10, wherein the sleeve insertion tool further includes one or more longitudinal slots defined in the body and extending from the proximal end toward the distal end, the method further comprising removing the sleeve insertion tool by peeling the sleeve insertion tool off the distal end of the surgical tool along the one or more longitudinal slots.

12. The method of any one of claims 8 to 11, wherein the sleeve insertion tool further comprises a sleeve stop defined within the inner chamber, the method further comprising engaging the distal end of the protective sleeve against the sleeve stop when the protective sleeve is introduced into the inner chamber.

13. The method of any one of claims 8 to 12, wherein the sleeve insertion tool further comprises a cap positioned within the jaw cavity, the method further comprises engaging the one or more jaw members against the cap while the sleeve insertion tool is advanced proximally relative to the surgical tool.

14. The method of claim 13 or the sleeve insertion tool of claim 7, wherein the cap comprises a rigid material selected from the group consisting of a metal, a plastic, a composite material, or any combination thereof.

15. The method of any one of claims 8 to 14, wherein positioning the sleeve insertion tool proximate the distal end of a surgical tool is preceded by introducing the protective sleeve at least partially into the inner chamber.

16. The method of any one of claims 8 to 15, wherein positioning the sleeve insertion tool proximate the distal end of a surgical tool is preceded by receiving the sleeve insertion tool with the protective sleeve pre-assembled within the inner chamber.

17. The method of any one of claims 8 to 16, further comprising protecting a user from accidental cuts caused by the one or more jaw members with the sleeve insertion tool.
